# EUROPÄISCHE PATENTSCHRIFT

(11) **EP 0 941 466 B1**
(45) Veröffentlichungstag und Bekanntmachung des Hinweises auf die Patenterteilung: **22.10.2003**
(21) Anmeldenummer: 97952809.8
(22) Anmeldetag: 26.11.1997
(51) Int. Cl.: G01N 15/02, G01N 21/64

(54) **VERFAHREN UND ANORDNUNG ZUM BESTIMMEN VORGEGEBENER EIGENSCHAFTEN VON ZIELPARTIKELN EINES PROBENMEDIUMS**
METHOD AND DEVICE FOR DETERMINING PREDETERMINED PROPERTIES OF TARGET PARTICLES OF A SAMPLE MEDIUM
PROCEDE ET DISPOSITIF POUR DETERMINER DES CARACTERISTIQUES PREDEFINIES DE PARTICULES CIBLES D'UN MILIEU D'ECHANTILLONNAGE

(30) Priorität: 27.11.1996 DE 19649048; 28.01.1997 DE 19702914
(43) Veröffentlichungstag der Anmeldung: 15.09.1999
(73) Patentinhaber: Max-Planck-Gesellschaft zur Förderung der Wissenschaften e.V. Berlin, 80539 München (DE); Evotec OAI AG, 22525 Hamburg (DE)
(72) Erfinder: SEIDEL, Claus, D-37085 Göttingen (DE); BRAND, Leif, D-25436 Tornesch (DE); GÜNTHER, Rolf, D-22525 Hamburg (DE)
(74) Vertreter: Schmidt, Frank-Michael, Dr.-Ing.
(86) Internationale Anmeldenummer: EP9706601
(87) Internationale Veröffentlichungsnummer: WO98023941

(56) Entgegenhaltungen:
- US-A- 5 434 667
- KELLER R A ET AL: "SINGLE-MOLECULE FLUORESCENCE ANALYSIS IN SOLUTION" APPLIED SPECTROSCOPY, Bd. 50, Nr. 7, Juli 1996, Seiten 12A-32A, XP000642337 in der Anmeldung erwähnt
- ZANDER C ET AL: "detection and characterization of single molecules in aqueous solution" APPLIED PHYSICS B: LASERS AND OPTICS., Bd. B63, Nr. 5, November 1996, BERLIN DE, Seiten 517-523, XP002066657 in der Anmeldung erwähnt
- BECKER W ET AL: "FLEXIBLE INSTRUMENT FOR TIME-CORRELATED SINGLE-PHOTON COUNTING" REVIEW OF SCIENTIFIC INSTRUMENTS, Bd. 62, Nr. 12, 1.Dezember 1991, Seiten 2991-2996, XP000278564
- MUSOLINO M ET AL: "AN INTEGRATED INSTRUMENTATION FOR LIGHT-SCATTERING AND TIME-RESOLVED FLUORESCENCE MEASUREMENTS" REVIEW OF SCIENTIFIC INSTRUMENTS, Bd. 66, Nr. 3, 1.März 1995, Seiten 2405-2410, XP000529679

## Beschreibung

Die Erfindung bezieht sich auf Verfahren zum Bestimmen von Eigenschaften von Zielpartikeln eines Probenmediums sowie auf eine Anordnung zur Durchführung des Verfahrens.

Zeitkorreliertes Einzelphotonen-Zählen ist eine vielseitige spektroskopische Technik, die es erlaubt, eine Vielzahl von Parametern zu untersuchender Partikel zu bestimmen. Dazu zählen die Fluoreszenzlebensdauer, ggf. auch die verschiedenen Fluoreszenzlebensdauern von multiexponentiellen Zerfällen, Rotationsdiffusionskonstanten, kinetische Übergangsraten, u.ä. Außerdem erlaubt die mit dem zeitkorrelierten Einzelphotonen-Zählen verbundene hohe Zeitauflösung in Verbindung mit gepulster Anregung eine effektive Trennung von unverzögert gestreutem Anregungslicht und verzögertem Fluoreszenzlicht. Dadurch lassen sich das gesuchte Signal (Fluoreszenz) und unerwünschtes Rauschen (unverzögertes Streulicht) voneinander trennen und das Signal-Rausch-Verhältnis für viele Anwendungen erhöhen.

Weitere Eigenschaften, insbesondere die Diffusionskonstante von Zielpartikeln, lassen sich mit der Technik der Fluoreszenz-Korrelations-Spektroskopie (FCS) gewinnen. Die Kenntnis der Diffusionskonstante ermöglicht in vielfältiger Weise Aussagen über die Größe der Zielpartikel oder ihren Bindungszustand an andere, große Moleküle.

Bei herkömmlichen FCS-Meßverfahren (vgl. WO 94/16313) besteht jedoch keine Möglichkeit der Diskriminierung zwischen unverzögert gestreutem Anregungslicht und verzögertem Fluoreszenzlicht, so daß das Signal-Rausch-Verhältnis zu gering bleibt.

Beide Techniken, zeitkorreliertes Einzelphotonen-Zählen und Fluoreszenz-Korrelations-Spektroskopie, sind für hochempfindliche Messungen an stark verdünnten Lösungen fluoreszierender Partikel, vorzugsweise im subnanomolaren Bereich, geeignet. Es können mit diesen Techniken sogar einzelne Moleküle in Probenmedien detektiert werden (vgl. C. Zander et al., Applied Physics B, Band 63, S. 517-523, 1996). Allerdings kann bisher die Autokorrelationsfunktion von Signalen einzelner Partikel nicht bestimmt werden.

Ein Verfahren und eine Anordnung der eingangs genannten Art wurde von Richard A. Keller et al. in Applied Spectroscopy, Band 50, Nr. 7, 1996, auf den Seiten 12A bis 32A beschrieben. Die Datenanalysemöglichkeiten sind bei der beschriebenen Anordnung auf ein isoliertes Anwenden entweder der Auswertemöglichkeiten des zeitkorrelierten Einzelphotonen-Zählens oder der FCS begrenzt. Die Vorteile beider Techniken werden nicht kombiniert genutzt. Ferner arbeitet die Auswerteeinrichtung der aus dieser Druckschrift bekannten Anordnung mit einem aufwendigen und kostspieligen CAMAC-Rahmen und benötigt einen Multi-Channel-Scaler (MCS).

Der Erfindung liegt die Aufgabe zugrunde, die Anwendungsmöglichkeiten der Fluoreszenz-Korrelations-Spektroskopie zu erweitern.

Diese Aufgabe wird erfindungsgemäß durch ein Verfahren mit den Merkmalen des Anspruchs 1 bzw. durch ein Verfahren mit den Merkmalen des Anspruchs 15 sowie durch eine Anordnung mit den Merkmalen des Anspruchs 18 gelöst.

Bei der ersten Ausführungsform des erfindungsgemäßen Verfahrens zum Bestimmen von Eigenschaften von Zielpartikeln eines Probenmediums wird das Probenmedium zunächst mit periodisch moduliertem Licht bestrahlt. Vorzugsweise wird das Probenmedium mit einer regelmäßigen Folge gleichmäßig beabstandeter Lichtimpulse bestrahlt. Der Abstand zwischen zwei Lichtimpulsen (die Periodendauer) beträgt beispielsweise 12ns. Anschließend wird im Probenmedium gestreutes Licht in Form von einzelnen Photonen von einer Detektionseinrichtung detektiert. Dabei wird der zeitliche Abstand zwischen dem Detektionszeitpunkt jedes Photons und einem in der zugehörigen Periode des bestrahlenden Lichts liegenden Referenzzeitpunkt als Verzögerungszeit registriert; zusätzlich wird der Detektionszeitpunkt jedes Photons registriert. Dies geschieht in der Regel mit zwei unterschiedlichen, auf die jeweilige Meßaufgabe abgestimmten Meßanordnungen. Die Verzögerungszeit, welche im Nanosekundenbereich liegt, erfordert eine analoge Meßanordnung zur Messung extrem kurzzeitiger Vorgänge. Die Bestimmung des Detektionszeitpunkts andererseits wird in der Regel mit einer digitalen Meßanordnung bestimmt, die geeignet ist, die entsprechenden Zeiten im Mikrosekundenbereich zu messen.

Aus den Verzögerungszeiten jeweils einer Anzahl unmittelbar aufeinanderfolgend detektierter Photonen wird wenigstens ein Parameter bestimmt. Die Anzahl von aufeinanderfolgend detektierten Photonen kann beispielsweise fest vorgegeben sein oder an das Vorhandensein von Zielpartikeln im Probenraum angepaßt werden. Ein erster Parameterwert wird für eine Anzahl von aufeinanderfolgend detektierten Photonen bestimmt. Daraufhin wird ein weiterer Parameterwert aus den Verzögerungszeiten einer weiteren Anzahl von aufeinanderfolgend detektierten Photonen bestimmt. Dabei kann die weitere Anzahl der aufeinanderfolgend detektierten Photonen einerseits sich an die vorhergehende Anzahl von detektierten Photonen anschließen, andererseits kann die zweite Anzahl von Photonen jeweils einen Teil der ersten Anzahl von Photonen mit umfassen (überlappen). Im ersten Fall ergäbe sich eine Aufteilung der aufeinanderfolgend detektierten Photonen in aufeinanderfolgende Gruppen, zu denen jeweils ein Parameterwert bestimmt wird. Im zweiten Fall wird praktisch ein über die aufeinanderfolgenden detektierten Photonen hinweggleitendes Auswahlzeitfenster gelegt, wobei die Verzögerungszeiten der in das Zeitfenster fallenden Photonen zur Bildung des Parameterwerts verwendet werden.

Um einen Parameterwert aus den Verzögerungszeiten zu bestimmen, werden in der Regel weitere Informationen herangezogen, die aus einer Vorauswertung bestimmter Partikeleigenschaften gewonnen wurden. Diese liegen beispielsweise als spektroskopische Daten vor, zum Beispiel in Form vorbekannter Fluoreszenzlebensdauern. Die Verzögerungszeiten für eine vorgegebene Anzahl von aufeinanderfolgend detektierten Photonen werden dann mit Hilfe der hinterlegten spektroskopischen Daten ausgewertet. Beispielsweise können dabei Amplitudenanteil unverzögerter Streulichtanteile und verzögerter Fluoreszenzlichtanteile quantitativ analysiert werden.

Mittels der Detektionszeitpunkte der jeweiligen Anzahl unmittelbar aufeinanderfolgend detektierter Photonen wird dann ein Zeitwert bestimmt. Anschließend werden mittels des wenigstens einen Parameters und des Zeitwerts der jeweiligen Anzahl unmittelbar aufeinanderfolgend detektierter Photonen jeweils Parameter-Zeit-Wertepaare bestimmt und aus den .Parameter-Zeit-Wertepaaren mehrerer aufeinanderfolgender Anzahlen wenigstens eine Parameter-Zeit-Funktion bestimmt. Dann wird eine Korrelationsfunktion der wenigstens einen Parameter-Zeit-Funktion berechnet. Die Korrelation der so gewonnenen Parameter-Zeit-Funktion gestattet erweiterte und neuartige Aussagen über Eigenschaften der Partikel.

Die erfindungsgemäße Anwendung der Korrelationstechnik auf die mit Hilfe von zeitkorreliertem Einzelphotonen-Zählen gewonnenen Parameterwerte erlaubt die Bestimmung neuartiger Eigenschaften der Zielpartikel und erweitert die Möglichkeiten der bekannten Techniken. So kann beispielsweise die Autokorrelationsfunktion des isolierten Amplitudenanteils des auf Fluoreszenz zurückzuführenden Anteils des gestreuten Lichts berechnet werden. Dadurch ergibt sich eine effektive Unterdrückung von Hintergrundsignalen. Aus derart berechneten Korrelationsfunktionen kann beispielsweise die Diffusionskonstante des Zielpartikels bestimmt werden. Gegenüber der herkömmlichen FCS führt das erfindungsgemäße Verfahren zu einer deutlichen Verbesserung des Signal-Rausch-Verhältnisses. In ultraverdünnten Lösungen werden durch das erfindungsgemäße Verfahren derartige Messungen überhaupt erst ermöglicht.

Bei einer vorteilhaften Weiterbildung des erfindungsgemäßen Verfahrens wird das im Probenmedium gestreute Licht von mehr als einem Detektor erfaßt. Anschließend wird jeweils wenigstens eine Parameter-Zeit-Funktion für die von jedem Detektor detektierten Photonen getrennt berechnet. Vorzugsweise wird anschließend als Korrelationsfunktion eine Kreuzkorrelationsfunktion mit Hilfe der Parameter-Zeit-Funktionen verschiedener Detektoren berechnet. Dies gestattet eine genaue Beobachtung der Bindungsreaktionen zwischen Partikeln, indem z.B. unterschiedliche Spektralfilter in den einzelnen Detektionszweigen eingesetzt werden.

Bei einer alternativen Ausführungsform des Verfahrens zum Bestimmen vorgegebener Eigenschaften von Zielpartikeln eines Probenmediums wird das Probenmedium zunächst mit Licht bestrahlt, dann im Probenmedium gestreutes Licht in Form von einzelnen Photonen von einer Detektionseinrichtung detektiert und der Detektionszeitpunkt jedes gestreuten Photons erfaßt. Die Dichte der Zielpartikel wird dabei so gewählt, daß sich im Mittel weniger als ein Zielpartikel in einem beobachteten Volumenelement des Probenmediums (Probenraum) befindet. Durch Bewerten der erfaßten Detektionszeitpunkte werden Zeitintervalle bestimmt, in denen Zielpartikel einzeln im Probenraum vorlagen. Dies geschieht beispielsweise durch Erfassen der zeitlichen Abstände zwischen aufeinanderfolgenden Detektionszeitpunkten, wobei der Eintritt eines Zielpartikels in den Probenraum dann erkannt wird, wenn z. B. eine vorgegebene Anzahl von zeitlichen Abständen einen Maximalabstand unterschreitet. Andererseits kann beispielsweise auch die Anzahl der erfaßten Detektionszeitpunkte in einem vorgegebenen Zeitintervall bewertet werden, wobei das Vorhandensein eines Zielpartikels im Probenraum dann angenommen wird, wenn die Anzahl der pro Zeiteinheit erfaßten Detektionszeitpunkte einen Minimalwert überschreitet. Aus den in einem aus den Zeitintervallen ausgewählten Zeitintervall erfaßten Detektionszeitpunkten wird wenigstens eine auf das ausgewählte Zeitintervall begrenzte, zeitabhängige Funktion gebildet, die eine Anzahl der pro vorgegebener Zeiteinheit erfaßten Detektionszeitpunkte angibt, wobei die vorgegebene Zeiteinheit wesentlich kürzer als das Zeitintervall gewählt wird.

Anschließend wird eine Korrelationsfunktion der zeitabhängigen Funktion berechnet. Dann werden die Eigenschaften der Zielpartikel mit Hilfe dieser Korrelationsfunktion bestimmt.

Im Unterschied zum Stand der Technik werden bei diesem erfindungsgemäßen Verfahren die erfaßten Detektionszeitpunkte selektiert, bevor eine mit Hilfe der Detektionszeitpunkte berechnete Detektionsfunktion einer Korrelation unterzogen wird. Die Detektionsfunktion kann beispielsweise eine Abtastfunktion sein, die den Wert Null annimmt, wenn in einem Abtastzeitintervall keine.Detektion eines Photons stattfand, und die den Wert Eins annimmt, wenn in einem Abtastzeitintervall eine Detektion stattfand. Bei einem größer gewählten Zeitintervall kann die Detektionsfunktion auch die Anzahl der in den jeweils konstanten Zeitintervallen erfaßten Detektionszeitpunkte wiedergeben.

Bei der erfindungsgemäßen Anordnung werden für sämtliche detektierten Photonen sowohl die zeitlichen Abstände zwischen den jeweiligen Detektionszeitpunkten der Photonen und einem Referenzzeitpunkt für die jeweilige Periode des bestrahlenden Lichts, also die Verzögerungszeiten, bestimmt, als auch mit Hilfe der Zähleranordnung die zeitlichen Abstände zwischen aufeinanderfolgenden Detektionszeitpunkten von Photonen. Beide Zeiten werden jeweils paarweise zugeordnet gespeichert.

Das Bestimmen des zeitlichen Abstands zwischen aufeinanderfolgenden Detektionszeitpunkten von Photonen geschieht erfindungsgemäß mit einer Wechselzähleranordnung, die mindestens einen ersten und einen zweiten Zähler enthält. Sie wird durch elektrische Impulse der Detektionseinrichtung gesteuert und ist so geschaltet, daß die Zähler abwechselnd zählen. Wenn also ein Photon detektiert wurde, startet der entsprechende Impuls den ersten Zähler. Wird ein zweites Photon detektiert, so stoppt der zugehörige elektrische Impuls der Detektionseinrichtung den ersten Zähler und startet den zweiten Zähler. Während der zweite Zähler zählt, kann der Zählerstand des ersten Zählers an die nachgeschaltete Recheneinheit übertragen werden. Bei Eintreffen eines dritten Photons wird der zweite Zähler gestoppt und der erste Zähler wieder gestartet. In der darauffolgenden Zeit kann der Zählerstand des zweiten Zählers ausgelesen und an die nachgeschaltete Recheneinheit übertragen werden, während der erste Zähler erneut zählt.

Ein Vorteil der erfindungsgemäßen Anordnung besteht darin, daß sie mit einfachsten konstruktiven Mitteln, nämlich Zählern und bekannten Schaltungselementen, realisiert werden kann.

Ein weiterer Vorteil besteht darin, daß die Anordnung totzeit- oder verlustlos arbeitet. Befindet sich ein Zähler in einem Reset-Vorgang, ist der andere Zähler zum Zählen bereit.

Schließlich besteht ein weiterer Vorteil der erfindungsgemäßen Anordnung darin, daß die mit ihr realisierbare Zeitauflösung für die Bestimmung der Detektionszeitpunkte der Photonen sehr hoch ist und flexibel vorgegeben werden kann.

Die Erfindung ermöglicht es, einzelne oder wenige Moleküle mit vertretbarem apparativen Aufwand, hoher Genauigkeit großer und Vielfalt der Analysemöglichkeiten spektroskopisch zu untersuchen. Durch die Erfindung gelingt es, das von den Zielpartikeln gestreute Licht wirksam von störenden Hintergrundsignalen zu trennen und als Folge davon die gesuchten Eigenschaften der Zielpartikel mit bisher nicht erreichter Genauigkeit zu erfassen.

Vorteilhafte Weiterbildungen der Erfindung sind in den Unteransprüchen gekennzeichnet.

Im folgenden wird die Erfindung anhand von Ausführungsbeispielen näher erläutert, die in den Figuren schematisch dargestellt sind. Gleiche Bezugsziffern in den einzelnen Figuren bezeichnen dabei gleiche Elemente. Im einzelnen zeigt:
- Fig. 1: eine schematische Darstellung eines ersten Ausführungsbeispiels der Erfindung;
- Fig. 2: eine schematische Darstellung eines zweiten Ausführungsbeispiels der Erfindung;
- Fig. 3: eine Kurve, die die zeitlichen Abstände Δt aufeinanderfolgender Detektionszeitpunkte von Photonen für eine Photonenfolge beschreibt;
- Fig. 4: ein Histogramm, bei dem die Anzahl der pro Zeiteinheit detektierten Photonen für die Photonenfolge der Fig. 3 aufgetragen ist;
- Fig. 5: ein Histogramm, gewonnen aus den zeitlichen Abständen zwischen Anregungsimpulsen und Detektionszeitpunkten der Photonen, für ein erstes in Fig. 3 und 4 markiertes Intervall;
- Fig. 6: ein Histogramm, gewonnen aus den zeitlichen Abständen zwischen Anregungsimpulsen und Detektionszeitpunkten der Photonen, für ein zweites, in Fig. 3 und 4 markiertes Intervall;
- Fig. 7: eine Kurvenschar, die ein Beispiel der Auswertung von aufgenommenen Daten zeigt.

Das erfindungsgemäße Verfahren sowie die erfindungsgemäße Anordnung sind vielfältig anwendbar.

Als Eigenschaft eines Zielpartikels kann z.B. seine Bindungsfähigkeit an ein anderes Molekül oder der Wert seiner Diffusionskonstante in einem bestimmten Probenmedium bestimmt werden. Unter Eigenschaften werden auch Zustände oder Zustandsänderungen der Zielpartikel verstanden. Mögliche Zustände der Zielpartikel wären demnach z.B. der gebundene bzw. der freie Zustand. Zustandsänderungen können sowohl Übergänge zwischen "gebunden" und "frei" sein, als auch Übergänge zwischen verschiedenen elektronischen Zuständen des Zielpartikels. Im einfachsten Fall kann dies der Übergang zwischen dem ersten angeregten Singulett und dem elektronischen Grundzustand sein, also eine Fluoreszenz. Der Kehrwert der zugehörigen Geschwindigkeitskonstanten ist die Fluoreszenzlebensdauer.

Zielpartikel sind Partikel, die spektroskopisch vermessen oder detektiert werden sollen. Sie befinden sich in oder auf den Probenmedien. Zielpartikel können dabei sowohl Moleküle als auch Molekülaggregate oder -komplexe sein. Ferner kann diese Technik auf Atome, mikrobiologische Objekte, wie Viren, Zellorganellen oder Zellen und Membranen, oder sonstige kleine Objekte, wie Latexkügelchen, u.ä., angewendet werden.

Das Probenmedium ist in der Regel ein flüssiges Medium, insbesondere ein Lösungsmittel für die Zielpartikel. Typische Probenmedien sind etwa organische Lösungsmittel, Wasser oder geeignete Pufferlösungen für biologische Untersuchungen, Blut oder Kulturmedien von Pilz- oder Bakterienkulturen. Das Probenmedium kann flüssig, fest oder gasförmig sein, homogen oder heterogen, d.h. aus verschiedenen Phasen zusammengesetzt sein. Es kann etwa aus zwei Phasen bestehen, beispielsweise einer Flüssigkeit oder einem Gas die bzw. das sich über einer Festkörper-Oberfläche befindet.

Der Probenraum ist ein durch die gattungsgemäße Anordnung beobachtetes Volumenelement des Probenmediums.

Das die Zielpartikel des Probenmediums im Probenraum bestrahlende Licht kann sowohl sichtbares Licht sein, als auch ultraviolettes oder infrarotes Licht. Im allgemeinsten Fall ist es elektromagnetische Strahlung, die durch die Zielpartikel gestreut werden kann.

Das Licht kann beliebig moduliert sein, solange es eine feste Periode aufweist. Insbesondere kann es impulsförmig oder sinusförmig mit einem geeigneten Offset moduliert sein.

Das Licht kann in vielfältiger Weise gestreut werden. Insbesondere kann das Licht elastisch oder inelastisch gestreut werden, d.h. unter Beibehaltung oder Änderung seiner Wellenlänge. Ferner kann es unverzögert oder verzögert gestreut werden. Zu den verzögerten, elastischen Streuungen gehört die Lumineszenz und darunter insbesondere die Fluoreszenz mit typischen Verzögerungszeiten von einigen Nanosekunden.

Die Detektionseinrichtung besteht bei den gattungsgemäßen Anordnungen in der Regel sowohl aus einem geeigneten optischen Aufbau, aus einem oder mehreren Detektoren und einer nachgeschalteten Elektronik zur Aufbereitung der Detektionssignale, in der Regel einschließlich einer Analog-Digital-Wandlung.

Die Auswerteeinrichtung besteht bei den gattungsgemäßen Anordnungen in der Regel aus einem Interface zur Aufnahme der Daten von der Detektionseinrichtung sowie aus einer Recheneinheit, die häufig ein Computer ist.

Die Detektionszeitpunkte der Photonen können sowohl durch eine absolute Bestimmung seit Beginn der Messung erhalten werden, als auch durch eine Bestimmung des zeitlichen Abstands zwischen den Detektionszeitpunkten aufeinanderfolgend detektierter Photonen. Die beiden Fälle können durch Differenzbildung oder durch Aufsummieren der zeitlichen Abstände ineinander überführt werden.

Zu bestimmende Parameter können alle Parameter sein, die man mit zeitkorreliertem Einzelphotonen-Zählen bestimmen kann. Dies sind u.a. Fluoreszenzlebensdauern, ggf. auch die verschiedenen Fluoreszenzlebensdauern von multiexponentiellen Zerfällen, Rotationsdiffusionskonstanten, kinetische Übergangsraten, Photonenanzahlen bzw. Gesamtamplituden oder Amplitudenanteile von einzelnen Anteilen des gestreuten Lichts, ferner auch jede Form von statistischen Abstandmaßen zwischen gemessenem und gesuchtem Signal, z.B. ein Kleinste-Quadrate-Abstand oder ein Informationsmaß, wie es in M. Köllner, Applied Optics, Band 32(6) (1993), S. 806-820, beschrieben ist.

Korrelationsfunktionen können sowohl Auto- als auch Kreuzkorrelationsfunktionen der bestimmten Parameter sein, z.B. die Autokorrelationsfunktionen von bestimmten Amplitudenanteilen oder die Kreuzkorrelationsfunktionen von bestimmten Fluoreszenzlebensdauern.

Im folgenden werden zunächst einige Ausführungsbeispiele der erfindungsgemäßen Anordnungen näher erläutert. Danach wird im Detail erklärt, wie die Eigenschaften der Zielpartikel aus mit Hilfe der erfindungsgemäßen Anordnungen gewonnenen Daten erhalten werden.

Betrachten wir zunächst den optischen Aufbau. Üblicherweise wird dieser in Form eines konfokalen oder nahfeldoptischen Aufbaus realisiert. Bei einem nahfeldoptischen Aufbau, der eine maximale Ortsauflösung aufweist, wird das Licht der Lichtquelle, die zum Bestrahlen oder Anregen der Zielpartikel dient, durch eine Öffnung auf das Probenmedium geworfen. Die Öffnung hat dabei einen Abstand von nur ca. 100nm zum Probenraum. Ferner ist der Durchmesser der Öffnung kleiner als die Wellenlänge des Anregungslichts. Dadurch erreicht man eine extreme lokale Begrenzung der Intensitätsverteilung des Anregungslichts um die Öffnung herum. Ein solcher nahfeldoptischer Aufbau ist besonders dazu geeignet, einzelne Zielpartikel gezielt anzufahren, die auf einer Oberfläche sitzen.

Ein konfokaler Aufbau, der eine maximale Empfindlichkeit aufweist, ist dazu geeignet, ein kleines Volumenelement in einem Probenmedium, d.h. den Probenraum, zu bestrahlen und zu beobachten. Typischerweise läßt man zu diesem Zweck die Zielpartikel in das Beobachtungsvolumen hinein diffundieren und aus dem Beobachtungsvolumen hinaus diffundieren. Ebenso können die Zielpartikel in einem Flußsystem oder auf andere Weise in den Probenraum geführt oder bewegt werden.

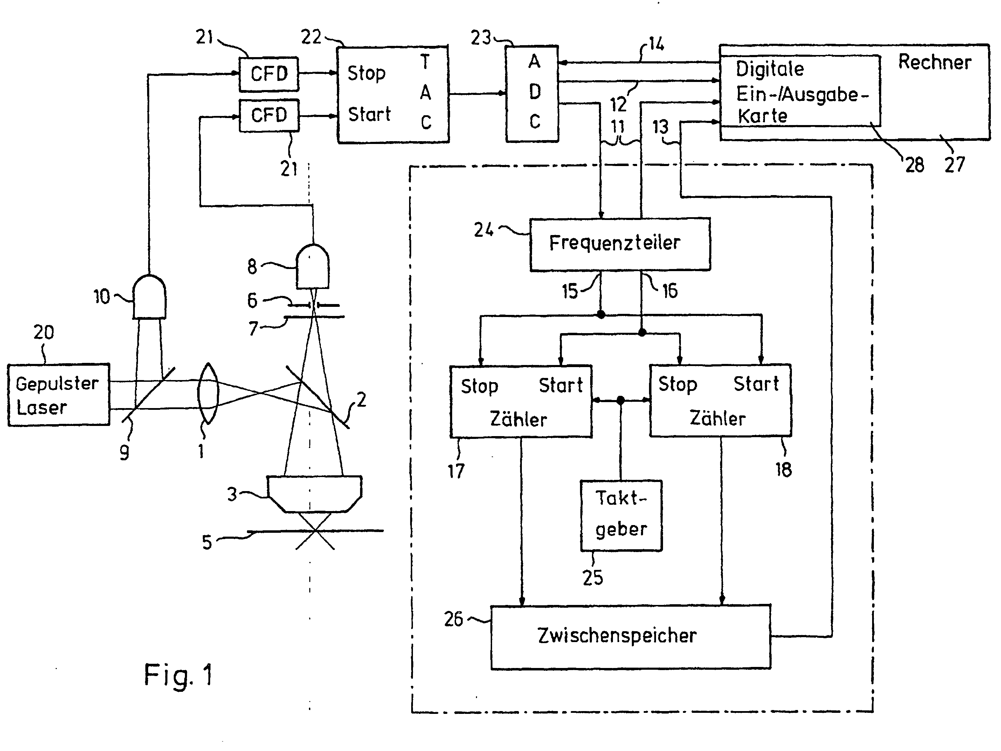

Im folgenden wird auf Fig. 1 Bezug genommen. Als Lichtquelle ist in Fig. 1 ein gepulster Laser 20 gezeigt. Typischerweise wird ein modengekoppelter Laser oder ein gepulster Diodenlaser verwendet, der Impulse mit einer Länge zwischen ca. 100fs (Femtosekunden) und ca. 500ps (Pikosekunden), je nach Art des Lasers, mit einem Abstand von ca. 10 bis 30ns (Nanosekunden) aussendet. Lichtquellen mit längeren Impulsen oder größeren Impulsabständen haben sich als ungünstig erwiesen.

Erfindungsgemäß kann statt einer gepulsten Lichtquelle auch eine periodisch modulierte Lichtquelle verwendet werden. Zur Analyse der Meßsignale wird dann sowohl die Amplitudenänderung als auch die Phasenänderung des Streulichts gegenüber dem Anregungslicht ausgewertet. Eine genauere Beschreibung dieses Meßprinzips findet sich in J.R. Lakowicz: "Principles of fluorescence spectroscopy", Plenum Press, New York, 1983.

Für den in Fig. 1 dargestellten konfokalen Aufbau wird das Laserlicht durch eine Linse 1 auf einen Punkt fokussiert. Im weiteren Verlauf des Strahlengangs wird das Laserlicht durch einen dichroitischen Umlenkspiegel 2 in ein Mikroskopobjektiv gelenkt. Der dichroitische Umlenkspiegel ist derart ausgebildet, daß er das Laserlicht reflektiert, jedoch das längerwellige Streu- bzw. Fluoreszenzlicht der Zielpartikel durchläßt. Mit Hilfe des Umlenkspiegels 2 und des Mikroskopobjektivs wird der Fokuspunkt des Lasers auf einen Punkt innerhalb des Probenmediums abgebildet. Das Probenmedium kann sich z.B. auf einen Probenhalter 5 in Form eines Objektträgers für die Mikroskopie befinden. Der vom Laser 20 beleuchtete Punkt innerhalb des Probenmediums wird mit Hilfe des Mikroskopobjektivs auf ein Pinhole 6 bzw. eine Lochblende abgebildet. Vor dem Pinhole befindet sich ein Spektralfilter 7, um gewünschte Streulichtanteile von ungewünschten farblich zu trennen. Direkt hinter dem Pinhole befindet sich ein Detektor 8, der so empfindlich ist, daß er einzelne Photonen nachweisen kann. Dazu eignen sich Photomultiplier, Mikrokanalplatten-Photomultiplier, Avalanche-Photodioden, CCDs oder CCDs mit vorgeschalteten Bildverstärkern. Eine genauere Beschreibung eines konfokalen Aufbaus findet sich in: G. Kalusche et al., Experimental Technique of Physics, Vol. 41(2) (1995) S. 265-276, bzw. in M. Eigen, R. Rigler, Proceedings of the National Academy of Sciences of the U.S.A., Vol. 91 (1994), S. 5740 - 5747.

Außer dem Signal des Photodetektors 8 wird noch ein Synchronisationssignal vom Laser 20 benötigt. Dies kann entweder als direktes elektrisches Signal von der Steuerelektronik des Lasers ausgegeben werden, oder es wird aus dem Laserlicht selbst abgeleitet. Dazu wird mit Hilfe eines Strahlteilers 9, der in den Strahlengang des Lasers 20 gestellt ist, ein Teil des Laserlichts auf einen Detektor 10 gelenkt. Dieser Detektor 10 kann z.B. eine Photodiode sein.

Um den Detektionszeitpunkt eines im Probenmedium gestreuten Photons zu bestimmen, muß der zeitliche Abstand zwischen dem Zeitpunkt der Anregung bzw. des Laserpulses und dem Zeitpunkt der Emission des Photons bestimmt werden. Handelt es sich bei dem gestreuten Licht um Fluoreszenz, so wird dieser zeitliche Abstand Fluoreszenz-Verzögerungszeit genannt. Er kann dadurch bestimmt werden, daß der zeitliche Abstand zwischen dem vom Detektor 10 ausgegebenen Triggerpuls und dem Detektionszeitpunkt eines Photons im Detektor 8 bestimmt wird.

Aufgrund des elektrischen und optischen Aufbaus wird es in der Regel eine konstante Verzögerung zwischen einem Triggerpuls des Detektors 10 und dem Zeitpunkt geben, an dem ein unverzögert gestreutes Photon vom Detektor 8 detektiert wird. Diese auch für alle sonstigen detektierten Photonen stets konstante Verzögerung kann durch eine geeignete Eichmessung und durch geeignete Verzögerungselemente ausgeglichen werden.

Um stets möglichen Jitter zu entfernen, werden die elektrischen Pulse der Detektoren 8 und 10 üblicherweise über sogenannte "constant fraction"-Diskriminatoren (CFD) 21 geleitet. Bei Wahl geeigneter Detektoren kann auf die CFDs 21 auch verzichtet werden.

Anschließend werden die Pulse einem sogenannten Zeit-zu-Amplituden-Konverter (**T**ime-to-**A**mplitude **C**onverter, TAC) 22 zugeführt. Ein TAC wandelt die Zeitdifferenz zwischen einem an ihm anliegenden Startpuls und einem an ihm anliegenden zeitlich verzögerten Stoppuls in eine Spannungsamplitude um. Der Startpuls startet eine im wesentlichen lineare Spannungsrampe, deren weitere Steigung bei Eintreffen des Stopppulses abgebrochen wird. Der bis dahin erreichte Spannungswert wird am Ausgang ausgegeben. Möchte man den zeitlichen Abstand zwischen dem Anregungspuls des Lasers und dem Detektionszeitpunkt des Photons bestimmen, so würde man zunächst das Triggersignal des Laserpulses dem Starteingang des TAC 22 zuführen und den elektrischen Puls des Detektors 8, der das Photon detektiert hat, dem Stoppuls zuführen (sog. normaler Modus). Bis auf konstante Verzögerungen könnte man somit den zeitlichen Abstand zwischen der Anregung des Zielpartikel und der Emission des Photons bestimmen.

Nicht jedes emittierte Photon aus dem Probenraum kann von dem Mikroskopobjektiv 3 und dem Detektor 8 detektiert werden. Dies ist nur für ca. 1% möglich. Daher gibt es wesentlich mehr Anregungspulse des Lasers 20 als detektierte Photonen. Um aber verläßliche Bestimmungen der Eigenschaften vornehmen zu können, braucht man möglichst viele detektierte Photonen. Die Anzahl der detektierten Photonen kann dabei zwischen z.B. 50 und z.B. 50 Millionen liegen. Entsprechend muß die Zahl der Anregungspulse noch höher sein.

Erstellt man aus den bestimmten Verzögerungszeiten ein entsprechendes Histogramm, so wird man den zeitlichen Abfall der Fluoreszenz als Kurvenverlauf des Histogramms rekonstruiert wiederfinden.

Um den TAC 22 nicht unnötig zu belasten und damit Totzeitverluste hervorzurufen, wird in der Regel der Puls des Detektors 8, der ein detektiertes Photon anzeigt, auf den Starteingang des TAC 22 und der Triggerpuls des Lasers 20 auf den Stoppeingang des TAC 22 gegeben (sog. invertierter Modus). Dies entspricht effektiv einer Umkehr der Zeitrichtung. Durch ein erneutes Umkehren der Zeitrichtung und eine eventuelle konstante Verschiebung der Zeitachse in der nachgeschalteten Recheneinheit kann das ursprüngliche Signal ohne weiteres rekonstruiert werden.

Die Ausgangsspannung des TACs 22 wird einem Analog-Digital-Wandler (ADC) 23 zugeführt. Der digitalisierte Amplitudenwert wird anschließend über eine Leitung 12 einer digitalen Ein-/Ausgabekarte 28 und damit einem Rechner 27 zugeführt. Dort wird in der Regel eine Vielkanalanalyse vorgenommen, um das Histogramm aufzubauen. Das Histogramm wird somit aus den einzelnen digitalisierten Amplitudenwerten aufgebaut, die jeweils die Verzögerungszeit eines detektierten Photons widerspiegeln.

Die bis hierhin geschilderte, bekannte Anordnung erlaubt es nur, die Verzögerungszeit eines detektierten Photons zu bestimmen. Sie erlaubt es nicht, den Detektionszeitpunkt des Photons absolut zu bestimmen, d.h. seit Beginn der Messung, bezogen etwa auf einen fixen Referenzzeitpunkt oder bezogen auf den Detektionszeitpunkt des letzten detektierten Photons. Um dies zu ermöglichen, sind der bisher beschriebenen Anordnung verschiedene Komponenten hinzugefügt worden, die im folgenden näher erläutert werden.

In einem ersten Ausführungsbeispiel wird ein vom ADC 23 nach jeder Wandlung ausgegebener Anfragepuls über eine Leitung 11 zunächst an eine Umschalteinrichtung 24 geleitet und von dort weiter an die digitale Ein-/Ausgabekarte 28 zum Triggern einer Datenaufnahme. Der Anfragepuls bewirkt ein Umschalten der Ausgänge der Umschalteinrichtung 24, die so ausgebildet ist, daß sie auf der ansteigenden Flanke des Anfragepulses umschaltet und damit effektiv die Frequenz des Anfragepulses halbiert. Sie bildet somit eine Frequenzteilerschaltung. Die Frequenzteilerschaltung 24 basiert auf einem Flip-Flop und weist zwei Ausgänge auf, die in üblicher Weise gegeneinander negiert sind. Sie kann in bekannter Weise aus D- oder JK-Flip-Flops aufgebaut werden.

Der erste Ausgang 15 des Frequenzteilers 24 ist z.B. mit dem Stopeingang des ersten Zählers 17 und dem Starteingang des zweiten Zählers 18 gekoppelt. Entsprechend ist der zweite Ausgang 16 des Frequenzteilers 24 mit dem Starteingang des ersten Zählers 17 und dem Stopeingang des zweiten Zählers 18 gekoppelt. Eine umgekehrte Beschaltung wäre ebenso geeignet.

Die Frequenzteilerschaltung 24 möge nach Empfangen eines Anfragepulses vom ADC 23 z.B. den ersten Zähler 17 starten und den zweiten Zähler 18 stoppen. Der erste Zähler zählt daraufhin Takte eines Taktgebers 25, der in Fig. 1 gezeigt ist.

Der Taktgeber 25 kann z.B. eine gewöhnliche Quarzuhr sein, die durch Stecken gewisser Jumper in ihrer Frequenz variierbar ist. Typische Frequenzen für den Taktgeber 25 liegen zwischen 100 MHz und 20 kHz. Auch das vom Laser 20 selbst abgeleitete Triggersignal aus dem Detektor 10 kann als Takt verwendet werden.

Nach dem Detektieren eines weiteren Photons durch den Detektor 8 wird ein erneuter Anfragepuls durch den ADC 23 ausgelöst. Der Frequenzteiler 24 schaltet daraufhin seine Ausgänge um und der erste Zähler wird gestoppt und der zweite Zähler gestartet. Der gestoppte erste Zähler überschreibt daraufhin mit seinem Zählerstand einen Zwischenspeicher 26. Der Anfragepuls wird vom Frequenzteiler 24 über eine zweite Leitung 11 an die digitale Ein-/Ausgabekarte 28 weitergeleitet. Ebenso wird der Inhalt des Zwischenspeichers 26 über eine Leitung 13 an die Ein-/Ausgabekarte 28 weitergeleitet.

Durch Empfangen des Anfragepulses ist ferner die digitale Ein-/Ausgabekarte 28 bereit, Daten aufzunehmen. Sie nimmt daraufhin sowohl den vom ADC 23 ausgegebenen gewandelten TAC-Wert über die Leitung 12 als auch den Inhalt des Zwischenspeichers 26 über die Leitung 13 auf und speichert sie zusammen im Rechner 27.

Typischerweise hat der gewandelte TAC-Wert eine Breite von 8 Bit und der Zwischenspeicher 26 eine Breite von 24 Bit, so daß die digitale Ein-/Ausgabekarte 28 eine Eingangsbreite von 32 Bit aufweisen sollte. Andere Bitbreiten sind selbstverständlich auch geeignet, sofern die Frequenz des Taktgebers 25 und die Breite des Zwischenspeichers 26 an die experimentellen Gegebenheiten angepaßt sind. Auch der Einsatz von 64 Bit aufnehmenden Ein-/Ausgabekarten ist möglich. Dabei könnten verschiedene Bitbereiche für die Signale von unterschiedlichen Detektoren reserviert werden. Ebenso könnten einzelne Bits dazu dienen zu kodieren, welcher Detektor das jeweilige Photon detektiert hat.

Nach Beendigung der Datenaufnahme sendet die Ein-/Ausgabekarte 28 ein Bestätigungssignal über eine Leitung 14 an den Analog-Digital-Wandler, der daraufhin für eine erneute Wandlung bereit steht.

Wird ein drittes Photon vom Detektor 8 detektiert, so sendet der ADC 23 einen erneuten Anfragepuls über die Leitung 11 aus und der Frequenzteiler 24 schaltet seine Ausgänge erneut um. Daraufhin wird der erste Zähler erneut gestartet und der zweite Zähler gestoppt. Der zweite Zähler überschreibt daraufhin den Inhalt des Zwischenspeichers 26 und dieser Inhalt wird von der Ein-/Ausgabekarte 28 über die Leitung 13 aufgenommen. Gleiches geschieht für alle folgenden Photonen-Detektionsereignisse.

Auf diese Weise wird zu jedem Photon sowohl der zeitliche Abstand zum nachfolgenden Anregungspuls als auch der zeitliche Abstand zum vorhergehenden Detektionszeitpunkt eines Photons gespeichert.

Beide Informationen sind sowohl zusammen (siehe das erfindungsgemäße Verfahren weiter unten) als auch getrennt zur Auswertung geeignet. Interessiert man sich z.B. nur für den Detektionszeitpunkt der Photonen, so kann man die digitalisierten TAC-Werte vernachlässigen und die Detektionszeitpunkte einer geeigneten Auswertung zuführen. Interessiert man sich andererseits nur für den zeitlichen Verlauf des Fluoreszenzabklingens, so können die gewandelten TAC-Werte zu einem Histogramm zusammengefaßt und ausgewertet werden, ohne die zeitlichen Abstände der einzelnen Detektionszeitpunkte der Photonen weiter zu betrachten.

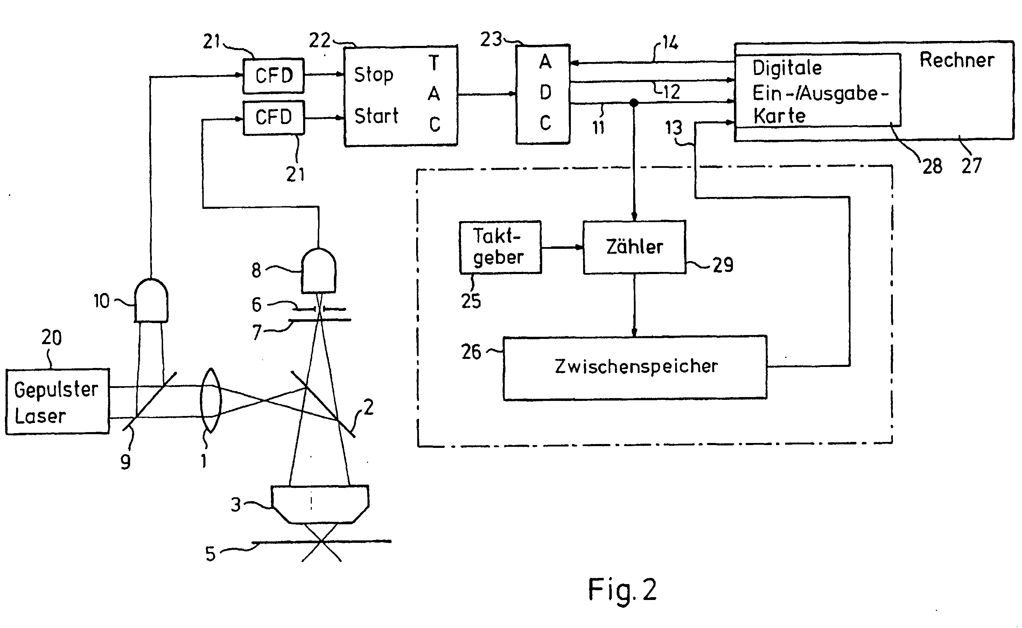

In einem zweiten Ausführungsbeispiel wird statt einer Wechselzähleranordnung nur ein einziger Zähler bzw. eine Uhr verwendet, wie es in Fig. 2 gezeigt ist, auf die im folgenden Bezug genommen wird.

Ein Anfragepuls des ADCs 23, der über die Leitung 11 ausgegeben wird, bewirkt, daß der in Fig. 2 gezeigte Zwischenspeicher 26 den Zeitwert des Zählers 29 bzw. der Uhr detektiert und zwischenspeichert. Der sich daraus ergebende Inhalt des Zwischenspeichers 26 wird von der digitalen Ein-/Ausgabekarte 28 nach Empfangen des Anfragepulses über eine Leitung 13 in gleicher Weise aufgenommen, wie es im ersten Ausführungsbeispiel beschrieben wurde.

In einem dritten Ausführungsbeispiel ist der Zähler 29 gemäß dem zweiten Ausführungsbeispiel derart geschaltet, daß ein Anfragepuls des ADCs 23 sein Anhalten bewirkt. Der aktuelle Zählerstand wird in den Zwischenspeicher 26 geschrieben, der Zähler 29 wird zurückgesetzt und beginnt erneut zu zählen. Der Inhalt des Zwischenspeichers 26 wird von der digitalen Ein-/Ausgabekarte 28 zusammen mit dem digitalisierten TAC-Wert über die Leitung 12 aufgenommen, nachdem die digitale Ein-/Ausgabekarte 28 den Anfragepuls des ADCs 23 über die Leitung 11 empfangen hat, wie es bereits im ersten Ausführungsbeispiel beschrieben wurde. Die Bitbreite des Zwischenspeichers 26 und des ADC-Werts kann in ähnlicher Weise, wie im ersten Ausführungsbeispiel beschrieben, ausgebildet sein.

Auch in diesem Ausführungsbeispiel zählt der Zähler 29 Takte eines z.B. in Fig. 2 gezeigten Taktgebers 25, der, wie im ersten Ausführungsbeispiel, in seiner Taktrate variabel ausgebildet sein kann.

Die in den Ausführungsbeispielen geschilderten Anordnungen können sowohl derart angeordnet werden, daß die Zähler, Zwischenspeicher, Taktgeber und Uhren sich außerhalb des Rechners 27 befinden und die Daten mit Hilfe der digitalen Ein-/Ausgabekarte 28 in den Rechner 27 aufgenommen werden. Ebenso gut können aber sämtliche Komponenten der Anordnungen auf einer gedruckten Leiterplatine angeordnet und in den Rechner 27 integriert werden.

In einem vierten Ausführungsbeispiel werden die Pulse der Detektoren 8 und 10 bzw. der CFDs 21 nicht durch einen TAC 22, einen ADC 23 und die beschriebenen weiteren Komponenten effektiv für den Rechner 27 aufbereitet. Vielmehr werden die Signale in ihrem analogen zeitlichen Verlauf mit möglichst hoher zeitlicher Genauigkeit detektiert. Dazu kann z.B. ein Zweistrahl-Oszilloskop mit möglichst großer Speichertiefe verwendet werden. Die so gewonnen Daten können anschließend wieder zu Verzögerungszeiten und Abständen zwischen Detektionszeitpunkten umgerechnet werden.

Nach der obigen Beschreibung der erfindungsgemäßen Anordnungen soll im folgenden beschrieben werden, wie aus den gewonnenen Daten die Eigenschaften der Zielpartikel bestimmt werden.

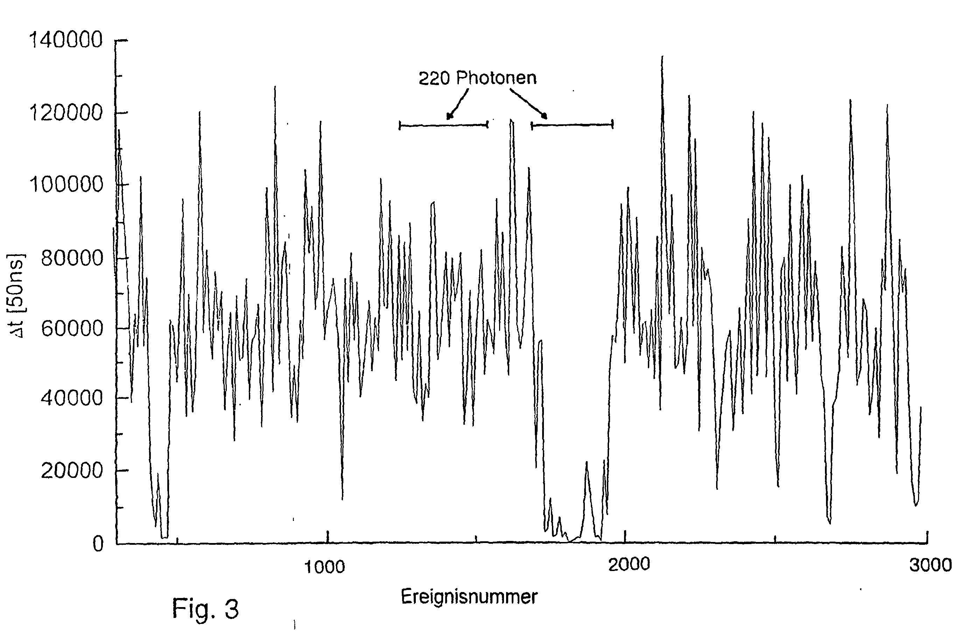

Im folgenden wird auf Fig. 3 bezug genommen. Fig. 3 zeigt eine Kurve, die die zeitlichen Abstände Δt aufeinanderfolgender Detektionszeitpunkte von Photonen für eine Photonenfolge beschreibt. Die Photonen sind dabei in der Reihenfolge ihrer Detektion durchnumerieret und auf der X-Achse mit ihrer Ereignisnummer bezeichnet. Der zeitliche Abstand zwischen aufeinanderfolgenden Detektionszeitpunkten von Photonen ist hier mit einer Genauigkeit von 50 Nanosekunden detektiert, was einer Taktrate des Taktgebers 25 von 20 MHz entspricht. Diese Messung wurde mit einem konfokalen Aufbau durchgeführt. Als Zielpartikel wurden Rhodamin-110 Farbstoff-Moleküle verwendet, die in Ethylenglykol gelöst wurden.

Befindet sich kein Zielpartikel bzw. Rhodamin-110 Molekül im Probenraum, ist die Zählrate der detektierten Photonen wesentlich geringer, als wenn ein stark absorptions- und fluoreszenzfähiges Farbstoff-Molekül wie Rhodamin-110 sich im beobachteten Volumenelement befindet. Beispiele dafür sind in Fig. 3 zu sehen. Um die Ereignisnummer von ca. 1.500 herum beträgt der zeitliche Abstand zwischen einzelnen Photonendetektionsereignissen ca. 60.000 x 50ns, also ca. 3ms, was einer Hintergrundzählrate von ca. 300Hz entspricht. Bei der Ereignisnummer von ca. 1.900 ist der zeitliche Abstand zwischen einzelnen Detektionszeitpunkten von Photonen wesentlich geringer. Der mittlere Abstand zwischen den einzelnen Photonenereignissen beträgt dort teilweise ca. 1.000 x 50ns, also ca. 50µs. Dies entspricht einer Detektionsrate von ca. 20kHz. Sie ist damit ca. 60 mal größer als die Hintergrundzählrate.

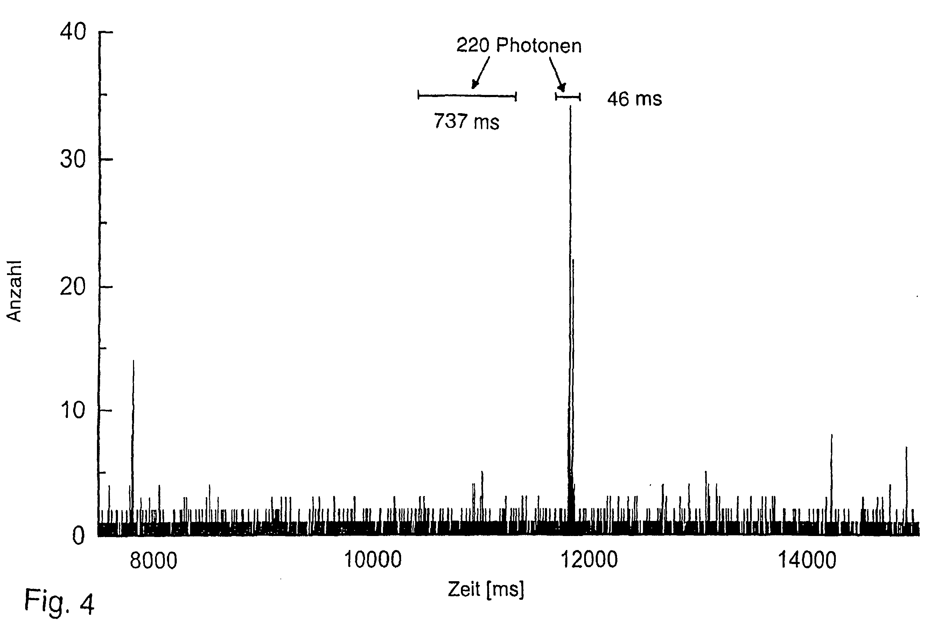

Dies wird unmittelbar einsichtig mit Hilfe der in Fig. 4 gezeigten Darstellung, auf die im folgenden Bezug genommen wird. Fig. 4 zeigt die Daten der Fig. 3, wobei auf der Y-Achse nicht mehr der zeitliche Abstand zwischen aufeinanderfolgenden Detektionszeitpunkten von Photonen aufgetragen ist, sondern die Anzahl der pro Millisekunde detektierten Photonen. Ferner ist die Ereignisnummer auf der X-Achse durch die Zeit ersetzt. Fig. 4 läßt sich aus den der Fig. 3 zugrundeliegenden Daten z.B. dadurch erhalten, daß die zeitlichen Abstände zwischen den einzelnen Detektionszeitpunkten aufsummiert werden und somit jedem Photon der absolute Zeitpunkt seiner Detektion zugeordnet werden kann. Sodann kann die Anzahl der in jedes Millisekunden-Intervall fallenden Detektionszeitpunkte bestimmt und aufgetragen werden. In Fig. 3 waren zwei Bereiche, die jeweils 220 detektierten Photonen entsprachen, markiert. Die gleichen Bereiche sind in Fig. 4 aufgetragen. Der Bereich um die Ereignisnummer von ca. 1.500 der Fig. 3 nimmt eine Zeitspanne von 737ms in Fig. 4 ein. Die gleiche Anzahl von 220 Photonen um die Ereignisnummer von 1.900 in Fig. 3, der eine wesentlich höhere mittlere Detektionsrate entsprach, nimmt in Fig. 4 nur eine Zeitspanne von 46ms ein. Man sieht, wie sich die Rate der Photonendetektion dadurch erhöht, daß sich ein Rhodamin-110 Molekül im Fokus bzw. Probenraum befindet. Es kommt in der Auftragung der Fig. 4 zu einem Peak der sich deutlich gegen den Untergrund abhebt.

Die Darstellungsweise der Fig. 4 wird gewöhnlich als Multi-Channel Scaling (MCS) oder Vielkanalzählung bezeichnet.

Während ein Meßverfahren, welches Daten in der Form der Fig. 4 erzeugt (MCS-Meßverfahren), das Nachweisen eines einzelnen Moleküls anschaulich macht, bietet ein Meßverfahren, das Daten in der Form der Fig. 3 aufnimmt, eine genauere Möglichkeit, die Daten auszuwerten. Dies liegt darin begründet, daß in Fig. 3 jedem einzelnen Photon sein genauer Detektionszeitpunkt zugeordnet werden kann. Bei MCS-Meßverfahren wird der Detektionszeitpunkt eines Photons hingegen häufig nur mit einer Genauigkeit von 1ms ermittelt.

Typische Durchmesser des Probenraums bzw. Fokus in einem konfokalen Aufbau betragen 0,5-1µm. Die Zeit, die ein Rhodamin-110 Molekül benötigt, um eine Distanz von 0,5µm zu diffundieren, d.h. durch den Fokus hindurch zu treten, beträgt im quadratischen Mittel ca. 500µs. Eine Kanalbreite der Integrationszeit von einer Millisekunde liegt daher nahe an der Durchtrittszeit der Moleküle durch den Fokus. Genauere Betrachtungen der Ein- und Austrittszeiten sind daher mit MCS-Daten nicht möglich. Solche Daten benötigt man aber, um Korrelationsfunktionen für die aufgenommenen Daten zu berechnen. Dies wird weiter unten im Detail geschildert.

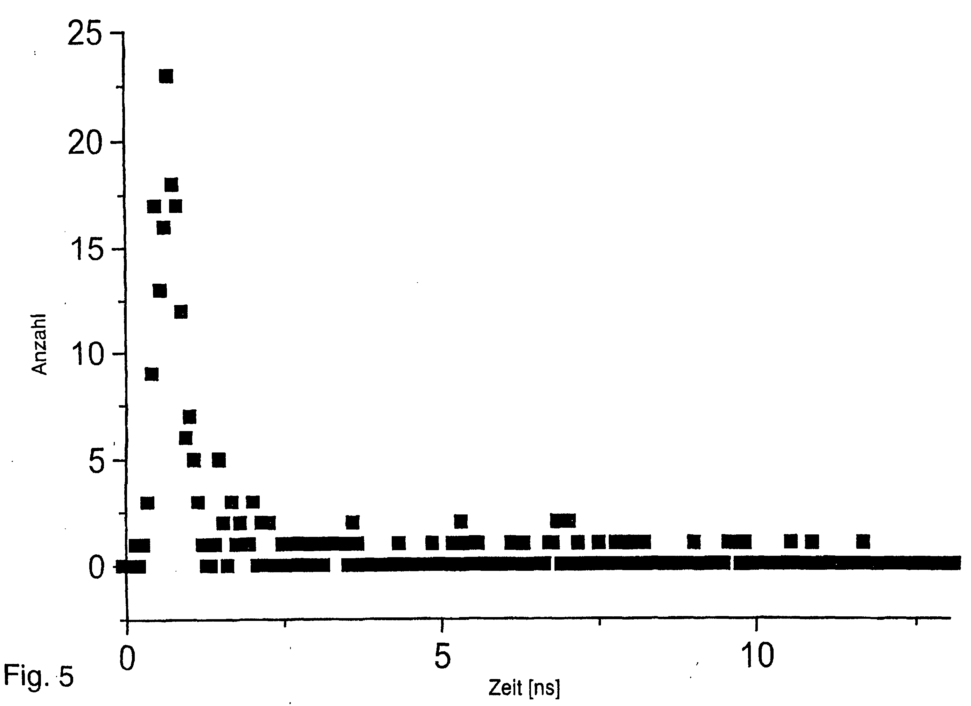

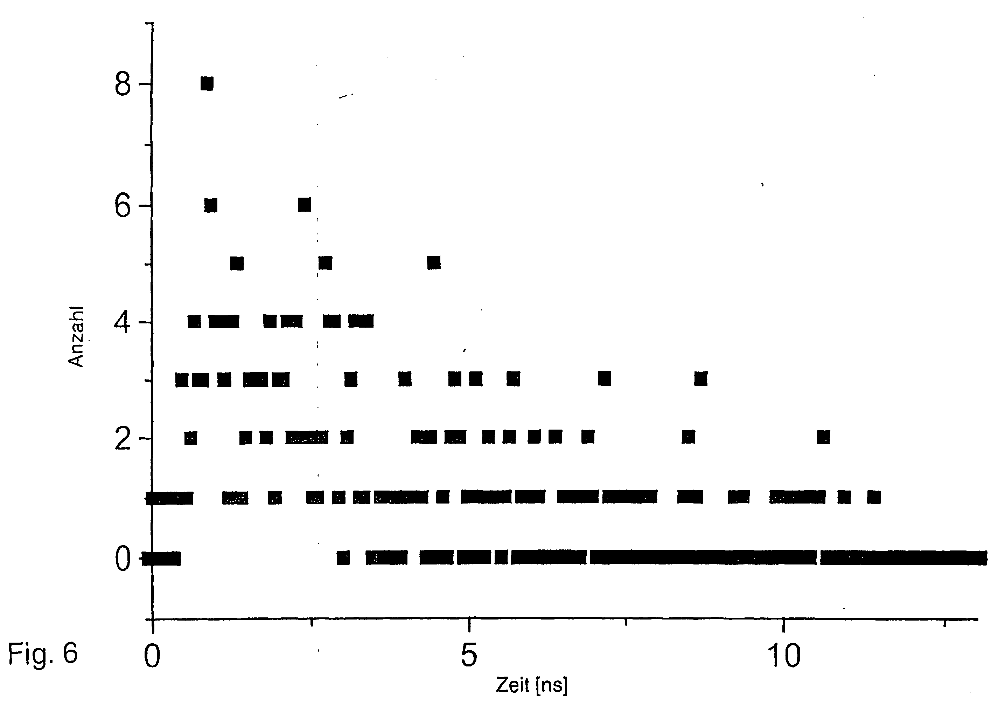

Für die Entscheidung, ob sich ein Zielpartikel während eines Meßintervalls im Fokus befand oder nicht, kann außer den in Fig. 3 bzw. Fig. 4 dargestellten Detektionsraten der Photonen auch das jeweilige Abklingverhalten des detektierten Signals auf einer ns-Skala für die einzelnen Meßintervalle betrachtet werden. Dabei wird, wie es für zeitkorreliertes Einzelphotonenzählen üblich ist, der zeitliche Abstand zwischen dem Laserpuls bzw. Anregungszeitpunkt und dem Detektionszeitpunkt der Photonen, also die Fluoreszenz-Verzögerungszeit, für die einzelnen Photonen des Meßintervalls in Histogrammform dargestellt. Dies ist in den Fig. 5 und 6 für Beispieldaten aus den Figuren 3 und 4 gezeigt.

Die Histogramme der Figuren 5 und 6 sind mit einer zeitlichen Auflösung von ca. 50 Pikosekunden/pro Kanal erstellt worden.

Zu jedem Photon wurde außer dem zeitlichen Abstand zum vorausgegangenen Detektionszeitpunkts eines Photons auch die zugehörige Verzögerungszeit gespeichert. Diese Werte liegen stets unterhalb der Periodendauer des Anregungslichts bzw. des zeitlichen Abstandes zwischen aufeinanderfolgenden Laserpulsen. In den Fig. 5 und 6 liegt die Verzögerungszeit zwischen 0 und ca. 12ns.

Fig. 5 zeigt ein Histogramm dieses zeitlichen Abklingverhaltens für den in Fig. 3 markierten Bereich von 220 Photonen um die Ereignisnummer von ca. 1.500 bzw. den in Fig. 4 markierten Bereich von 220 Photonen entsprechend 737ms. Wie aus den Figuren 3 und 4 deutlich wird, befindet sich während dieses Meßintervalls kein Zielpartikel im Probenraum. Entsprechend zeigt Fig. 5 einen zeitlichen Verlauf des Histogramms, der einen Peak zu einem frühen Zeitpunkt hat und danach einen fast gleichverteilten zeitlichen Verlauf annimmt. Der Peak entspricht unverzögertem Streulicht und der fast gleichverteilte weitere Verlauf entspricht sonstigem Hintergrundrauschen.

Im Gegensatz dazu zeigt Fig. 6 den zeitlichen verlauf der Fluoreszenz eines Rhodamin-110 Moleküls bzw. eines Zielpartikels. Fig. 6 zeigt ein Histogramm von der Art der Fig. 5 für den in Fig. 3 markierten Bereich von 220 Photonen um die Ereignisnummer von ca. 1.900 herum bzw. für den in Fig. 4 markierten Bereich von 220 Photonen entsprechend 46ms. Aus den Figuren 3 und 4 wurde bereits deutlich, daß sich in diesem Meßintervall ein Zielpartikel bzw. Rhodamin-110 Molekül im Fokus befindet. Entsprechend beobachtet man den in Fig. 6 gezeigten zeitlichen Verlauf der Fluoreszenz, der nach anfänglich steilem Ansteigen, was der Anregung durch den zeitlich kurzen Laserpuls entspricht, im wesentlichen gleichmäßig abfällt. Der zeitliche Abfall ist wesentlich langsamer als in Fig. 5. Die aus Fig. 6 zu erschließende Fluoreszenzlebensdauer liegt im Bereich von 3,6ns.

Zusätzlich sieht man in Fig. 6 zu Beginn einen deutlich ausgeprägten Peak, der von zusätzlichen unverzögert gestreuten Anteilen des gestreuten Lichts herrühren kann. Um zu entscheiden, ob der anfänglich Peak - und vergleichbare Peaks auf vergleichbaren Histogrammen - von zusätzlichen unverzögert gestreuten Anteilen des gestreuten Lichts herrührt, wird in gesonderten Messungen nur unverzögertes Streulicht aufgenommen, in dem z.B. reines Ethylenglykol vermessen wird. Man erhält daraus einen Fig. 5 vergleichbaren zeitlichen Verlauf, jedoch mit wesentlich höheren Anzahlen von detektierten Photonen pro Kanal des Histogramms.

Desgleichen würde man in gesonderten Messungen das Fluoreszenzabklingverhalten von Zielpartikeln, in diesem Fall Rhodamin-110, erfassen.

Mit Hilfe dieser dann vorbekannten Daten kann entschieden werden, wie groß der Anteil des unverzögert gestreuten Lichts zur Amplitude der Daten z.B. der Fig. 6 ist. Dies kann mit Hilfe von effizienten statistischen Algorithmen geschehen, etwa einer kleinsten Quadrate-Anpassung oder einer Maximum-Likelihood-Anpassung, die in der Literatur beschrieben wurden (siehe z.B.: J.N. Demas: "Excited state lifetime measurements", Academic Press, New York, 1983, bzw. M. Köllner, J. Wolfrum, Chemical Physics Letters, Band 200 (1992) S. 199 - 204).

Mit Hilfe eines Anpassungstests, der z.B. einen exponentiell abfallenden Verlauf für das Abklingen der Fluoreszenz des Zielpartikels und einen noch zu bestimmenden Anteil von unverzögertem Streulicht am Signal annimmt, kann durch die Berücksichtigung des Anteils des unverzögerten Streulichts die Bestimmung der Fluoreszenzlebensdauer mit größerer Exaktheit vorgenommen werden. In einer solchen Weise kann somit die Fluoreszenzlebensdauer eines Zielpartikels als vorgegebene Eigenschaft des Zielpartikels mit Hilfe der ermittelten Amplitudenanteile, in diesem Fall der Fluoreszenz und des unverzögertem Streulichts, bestimmt werden. Weitere Anteile etwa von anderen Partikeln oder anderen Streuquellen, können mit Hilfe von ähnlichen gesonderten Messungen und den daraus gewonnenen Daten berücksichtigt werden.

Um die Detektionseffizienz zu erhöhen, kann der in den Figuren 1 und 2 gezeigte optische Aufbau durch ein zweites Mikroskopobjektiv in Verlängerung des Strahlengangs des ersten Mikroskopobjektivs 3 unterhalb des Probenhalters 5 ergänzt werden. Die Lichtsammeleffizienz erhöht sich dadurch um einen Faktor 2. Dem Mikroskopobjektiv muß, wie dem Mikroskopobjektiv 3, ein Filter, ein Pinhole und ein Detektor nachgeschaltet werden. Die weitere nachgeschaltete Datenerfassungselektronik und -auswerteeinrichtung kann für den zweiten Detektionspfad gesondert aufgebaut werden, oder die Signale des zweiten Detektors werden dem bereits vorhandenen TAC 22 über eine Routingeinheit zugeführt. Im erstgenannten Falle einer gesonderten Datenerfassungsanordnung können die Photonen, die an den einzelnen Detektoren detektiert wurden, in der soeben geschilderten Weise einzeln ausgewertet werden. Insbesondere können die Amplituden der einzelnen Streulichtanteile, d.h. des verzögerten Streulichts bzw. der Fluoreszenz und des unverzögerten Streulichts, für die durch die einzelnen Detektoren detektierten Photonen bestimmt und daraus wiederum die Eigenschaften der Zielpartikel ermittelt werden. Ebenso können die Daten auch in den entsprechenden Histogrammen zusammengefaßt und gemeinsam ausgewertet werden.

Ferner kann die Detektionseffizienz durch den Einsatz eines geeigneten Spiegels direkt unterhalb der Probe erhöht werden.

Die Histogramme der Figuren 5 und 6 wurden für ein Meßintervall erstellt, in das 220 Photonen fielen. Ebenso gut hätte man eine beliebige andere sinnvolle Anzahl von Photonen zu einem Histogramm zusammenfassen können. Außerdem legt Fig. 4 es nahe, daß nicht für eine bestimmte Anzahl von Photonen sondern für ein bestimmtes vorgegebenes Zeitintervall die Histogramme wie in den Figuren 5 und 6 erstellt werden.

Für den Fall, daß die Intervalle durch eine vorgegebene Anzahl von detektierten Photonen definiert sind, kann eine gleitende Auswertung für verschiedene Photonen-Abschnitte der Fig. 3 dadurch vorgenommen werden, daß entsprechende Histogramme gebildet und analysiert werden. Z.B. kann jeweils ein später detektiertes Photon in das Histogramm aufgenommen und ein früher detektiertes Photon entfernt werden. Ebenso gut können statt einem Photon zehn oder eine beliebige andere Zahl von Photonen aufgenommen bzw. aus dem Histogramm entfernt werden. Ferner kann die vorgegebene Anzahl von detektierten Photonen auch variable gestaltet werden. Sie kann z.B. für den Fall einzeln vorliegender Zielpartikel größer oder kleiner gewählt werden, als für den Fall von sonstigem gestreuten Licht.

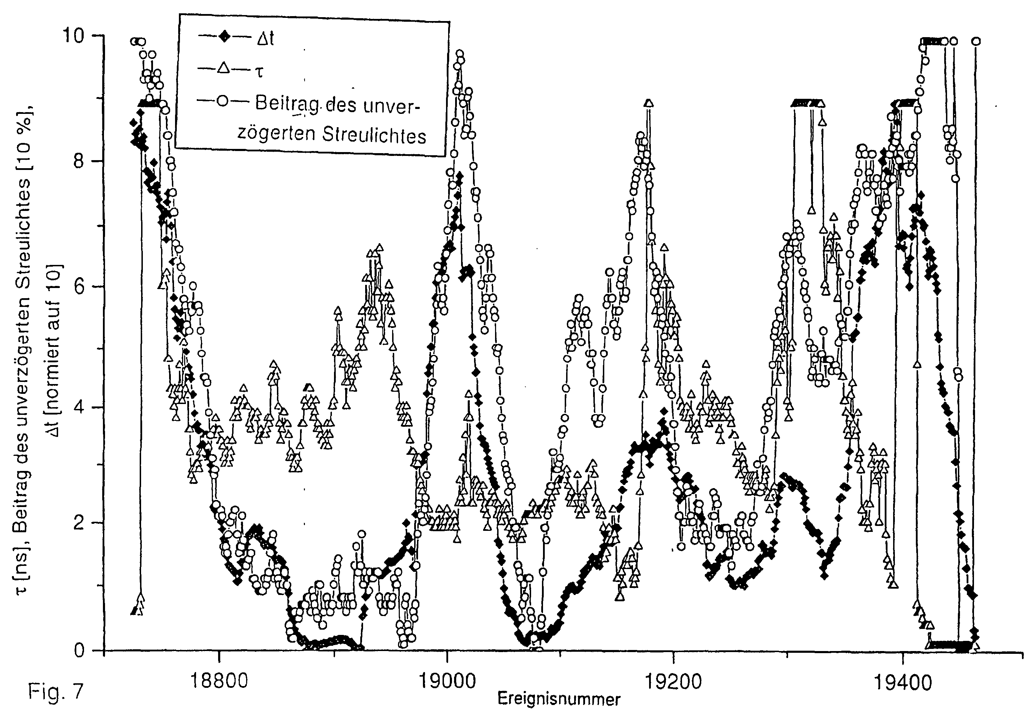

Die bestimmten Amplitudenanteile der einzelnen Streulichtanteile oder ggf. die bestimmte Lebensdauer können dann mit Hilfe Detektionszeitpunkte oder der zeitlichen Abstände zwischen aufeinanderfolgenden Detektionszeitpunkten als --Funktion der Zeit oder der Ereignisnummer aufgetragen werden. Dies ist beispielhaft in Fig. 7 gezeigt.

Fig. 7 zeigt eine geleitende Auswertung der Daten, die der Figur 3 zugrunde liegen, wobei die Histogramme jeweils auf der Basis von 40 Photonen erstellt wurden und das jeweils nächste Histogramm durch Hinzuziehen eines später detektierten Photons und Herauslassen eines früher detektierten Photons erstellt wurde. Auch der mit Δt bezeichnete Abstand zwischen den einzelnen Detektionszeitpunkten der Photonen, der in Fig. 7 durch "◆" gekennzeichnet ist, ist über jeweils 40 Photonen gemittelt.

Der Bereich um die Ereignisnummer von ca. 18.850 zeigt sehr deutlich, daß ein kleiner zeitlicher Abstand der detektierten Photonen, der auf das Vorhandensein eines Zielpartikels im Fokus hinweist, mit einem ebenso kleinen Amplitudenanteil des unverzögerten Streulichts und einer großen Fluoreszenzlebensdauer korreliert. Die Fluoreszenzlebensdauer beträgt in diesem Fall ca. 4 bis 5ns. Im Gegensatz dazu zeigt der Bereich um die Ereignisnummer von ca. 19.000 einen großen zeitlichen Abstand zwischen aufeinanderfolgenden Photonenereignissen, d.h. kein Zielpartikel befindet sich im Fokus, weshalb der Amplitudenanteil des unverzögerten Streulichts auf nahezu 100% ansteigt und die Fluoreszenzlebensdauer deutlich absinkt.

Daten von der Form, wie sie in Fig. 7 exemplarisch dargestellt ist, eignen sich erfindungsgemäß auch zur weiteren Verarbeitung. In der Literatur (z.B. M. Eigen, R. Rigler, Proeedings of the National Academy of Sciences of the U.S.A., Vol. 91 (1994), S. 5740 - 5747) ist allgemein beschrieben, daß mit Hilfe der Autokorrelationsfunktion der detektierten Photonen verläßlich bestimmt werden kann, ob nur ein Zielpartikel oder mehr als ein Zielpartikel sich während der Messung im Mittel im Fokus befand. Ferner kann die Diffusionskonstante eines Zielpartikels aus der Autokorrelationsfunktion des gestreuten Lichts ermittelt werden. Mit Hilfe von Korrelationsfunktionen kann auch das signifikante gemeinsame Vorliegen von einzelnen Anteilen des gestreuten Lichts bestimmt werden. Dazu berechnet man in bekannter Weise die Kreuzkorrelationsfunktion der einzelnen bestimmten Amplitudenanteile. Weist sie einen deutlichen Peak um den zeitlichen Nullpunkt auf, so liegen die betrachteten Streulichtanteil signifikant gleichzeitig im Signal vor bzw. treten in der Regel gleichzeitig auf. Dies erlaubt es, das gemeinsame Auftreten von zwei Zielpartikeln signifikant nachzuweisen.

Von Interesse ist eine solche Messung dort, wo für pharmakologische Zwecke die Bindung zweier Moleküle oder Molekülkomplexe aneinander festgestellt werden soll. Dies kann von Relevanz sein im sogenannten Drug Screening. Dort ist häufig relevant, eine besonders starke Bindung zwischen einem Zielpartikel, z.B. einem Antigen und einem anderen Zielpartikel, z.B. einem Antikörper, nachzuweisen. Sind die Zielpartikel, deren Bindung beobachtet werden soll, beide fluoreszenzmarkiert, so sollten die jeweiligen Amplitudenanteile gleichzeitig erhöhte Werte annehmen. Die Amplitudenanteile können dabei z.B. mit Hilfe der gesondert aufgenommenen Daten für diese Zielpartikel bestimmt werden. Ist die Bindung schwach, so wird dieses gleichzeitige Auftreten nur schwach ausgeprägt sein. Ist die Bindung stark, werden die Partikel in der Regel aneinander gebunden und daher gleichzeitig im Fokus beobachtet werden.

Bindung kann aber auch mit Hilfe eines Sandwich-Tests nachgewiesen werden. Bei einem Sandwich-Test wird die Bindung eines gesuchten Moleküls zwischen zwei anderen Molekülen beobachtet. Mindestens eines von diesen muß fluoreszierend oder fluoreszenzmarkiert sein. Sind beide fluoreszenzmarkiert, kann ihr gemeinsames Auftreten mit Hilfe einer Korrelationsfunktion bestimmt und zur quantitativen Analyse der Bindung herangezogen werden. Ist nur eines von ihnen fluoreszierend, kann die Bindung über eine geänderte Diffusionskonstante aus der Autokorrelationsfunktion erschlossen werden.

Da das erfindungsgemäße Verfahren die Untersuchung einzelner Partikel erlaubt, ermöglicht es, nicht nur den Mittelwert von physischen Meßwerten zu erfassen. Vielmehr kann auch die Verteilung des Meßwerts durch die Bestimmung vieler einzelner Meßwerte an einzelnen Zielpartikeln direkt ermittelt werden. Damit kann auch die Heterogenität einer heterogenen Menge von Zielpartikeln analysiert werden.

Erfindungsgemäß kann z.B. die Autokorrelationsfunktion der Fluoreszenzlebensdauern als bestimmter Parameter erstellt werden. Auch kann die Autokorrelationsfunktion des auf Fluoreszenz der Zielpartikel zurückgeführten Anteils am gestreuten Licht berechnet werden. Dieser Amplitudenanteil, kann dabei, wie bereits erklärt, unter Berücksichtigung der vorbekannten Fluoreszenzlebensdauer des Zielpartikels bestimmt werden. Daraus ergibt sich eine Fluoreszenzlebensdauer-selektive Autokorrelationsfunktion. Aus ihr kann z.B. eine zugehörige Diffusionskonstante ermittelt werden.

Im Gegensatz zu herkömmlichen FCS-Techniken kann die Korrelationsfunktion erfindungsgemäß selektiv und unter Eliminierung von Streulichtanteilen berechnet werden. Im Ergebnis ergibt sich ein verbessertes Signal-Rausch-Verhältnis bei extrem schwachem Signal, das sich aus selten und einzeln im Probenraum befindlichen Zielpartikeln ergibt.

Eine Fluoreszenzlebensdauer-selektive Autokorrelationsfunktion kann z.B. dazu verwendet werden, quantitativ die Bindung eines fluoreszierenden Zielpartikels zu analysieren, dessen Fluoreszenzlebensdauer sich im gebundenen und freien Zustand unterscheiden. Hierfür geeignete Farbstoffe sind z.B. die in der DE-OS 38 07 975 beschrieben sog. "intelligenten" Farbstoffe. Es kann hier erfindungsgemäß die Autokorrelationsfunktion selektiv für den gebundenen und den freien Zustand berechnet werden.

Es kann auch die Kreuzkorrelationsfunktion von unterschiedlichen Parametern berechnet werden, so daß sich signifikantes gemeinsames Auftreten oder signifikantes getrenntes Auftreten an einem Peak bzw. Tal um den zeitlichen Nullpunkt der Kreuzkorrelationsfunktion ablesen läßt.

Auch kann im Falle von zwei Detektoren die Kreuzkorrelationsfunktion von gleichen oder verschiedenen für die Daten der einzelnen Detektoren bestimmten Parameter berechnet werden. Auch hier lassen sich in gleicher Weise Aussagen über gemeinsames oder getrenntes Auftreten von Zielpartikeln ablesen.

Es kann auch vorkommen, daß sich mehr als ein Zielpartikel im Fokus befindet. Weisen verschiedene Zielpartikel unterschiedliche Fluoreszenzlebensdauern auf, so wird das zu einem Histogramm umgeformte Meßsignal einen multiexponentiellen zeitlichen Verlauf des Fluoreszenzabklingens aufweisen. Einen ähnlichen Verlauf kann die Fluoreszenz aufweisen, wenn das einzelne Zielpartikel bereits ein multiexponentielles Fluoreszenzabklingen zeigt. In einem solchen Fall können die weiter oben zitierten statistischen Verfahren dergestalt eingesetzt werden, daß für jeden einzelnen exponentiellen Zerfall ein gesonderter Amplitudenanteil bestimmt wird. Um dies sinnvoll durchführen zu können, werden wieder in gesonderten Messungen Daten über das Fluoreszenzabklingverhalten der verschiedenen Zielpartikel bzw. jedes einzelnen Zielpartikels aufgenommen.

Im Falle vom multiexponentiellen Fluoreszenzzerfällen bzw. im Falle des Vorliegens von mehr als einem Zielpartikel im Fokus können neben den Amplitudenanteilen der einzelnen Zielpartikel zum Gesamtstreulicht auch deren jeweilige Fluoreszenzlebensdauern bestimmt werden, sofern die verschiedenen Zielpartikel verschiedene Fluoreszenzlebensdauern aufweisen. Auch diese Amplitudenanteile oder Fluoreszenzlebensdauern können miteinander korreliert werden bzw. deren Kreuzkorrelationsfunktion kann berechnet werden. Das gemeinsame Auftreten von z.B. aneinander gebundenen Zielpartikeln kann durch ein gemeinsames Auftreten der erhöhten Amplitudenanteile bzw. Fluoreszenzlebensdauern nachgewiesen werden. Besonders geeignet sind dafür die in der DE 42 10 970 C2 beschriebenen sog. "Multiplex"-Farbstoffe.

Die spektroskopischen Daten können auch das Rotationsverhalten der Zielpartikel widerspiegeln, wie es in der Spektroskopie allgemein bekannt ist. Die Rotation eines Zielpartikels führt zu einer Depolarisation der Fluoreszenz während der Abklingzeit. Durch den Einsatz von polarisiertem Anregungslicht und einem Analysator vor dem Detektor 8 kann dieses Depolarisationsverhalten in einem Histogramm gemäß Fig. 6 bestimmt werden. Wird es in gesonderten Messungen genau bestimmt, kann ein Wissen darüber auch zur Bestimmung des Amplitudenanteils der Fluoreszenz eines Zielpartikels zum gesamten Streulicht verwendet werden.

Nebst spektroskopisch unabhängigen Fluorophoren für z.B. die zwei oben erwähnten Zielpartikel, können auch zwei Fluorophore verwendet werden, zwischen denen resonanter Energietransfer auftritt. So kann durch das Laserlicht das erste Zielpartikel angeregt werden, d.h. Laserlicht absorbieren, kann diese Energie resonant an das zweite Zielpartikel übertragen, und letzteres kann emittieren. Ein solcher resonanter Energietransfer ist stark abstandsabhängig. D.h. er wird dann effizient stattfinden, wenn die Zielpartikel aneinander gebunden sind und fast nicht auftreten, wenn die Zielpartikel nicht aneinander gebunden sind. Bestimmt man dann die Amplitudenanteile der einzelnen Zielpartikel am Streulicht und berechnet deren Kreuzkorrelationsfunktion, so wird sich eine starke Bindung zwischen den Zielpartikeln dahingehend äußern, daß nur die Fluoreszenz des zweiten, des die übertragene Energie aufnehmenden Zielpartikels zu beobachten sein wird, wenn beide gleichzeitig im Fokus vorliegen. Man wird also eine negative Korrelation in der Kreuzkorrelationsfunktion beobachten.

Der Verlauf einer Autokorrelationsfunktion für Streulicht, daß mit Hilfe eines oben beschriebenen konfokalen optischen Aufbaus aufgenommen wird, ist durch die Diffusion des fluoreszierenden Partikels bestimmt. Die charakteristischen Größen dafür sind einerseits die Diffusionskonstante des Partikels und andererseits die räumlichen Dimensionen des Fokus. Diese Diffusionskonstanten beeinflussen auch in entscheidender Weise die soeben beschriebenen Kreuzkorrelationsfunktionen. In separaten Messungen können die Diffusionskonstanten der einzelnen Zielpartikel bzw. der charakteristische Verlauf ihrer Autokorrelationsfunktion bestimmt werden. Mit Hilfe dieses Wissens können die Kreuzkorrelationen daraufhin ausgewertet werden, wie groß die Anteile der einzelnen Zielpartikel am Zustandekommen der Korrelationsfunktionen sind.

Die erfindungsgemäßen Anordnungen sind, wie bereits erwähnt, besonders dazu geeignet, einzelne Zielpartikel spektroskopisch zu untersuchen. Um zu entscheiden, ob bzw. daß ein einzelnes Zielpartikel im Probenraum oder -medium bzw. Beobachtungsvolumen vorliegt, müssen zunächst die Zielpartikel in einer solchen Verdünnung vorliegen, daß sich im Mittel weniger als ein Zielpartikel im beobachteten Teil des Probenmediums bzw. dem Probenraum befindet. Typische Konzentrationen hierfür sind 10⁻⁹-10⁻¹² M Lösungen (M = mol / Liter). Verwendet man z.B. eine 10⁻¹² M Lösung von Rhodamin-110 in Ethylenglykol und hat einen Fokus mit einer ellipsoiden Form, deren kurze Halbachsen 0,25µm und deren lange Halbachse 2,5µm betragen, so ist das Volumen des beobachteten Probenraums ca. 0,65µm³. Multiplikation mit einer Konzentration von 10⁻¹² mol / Liter an Rhodamin-110 Molekülen ergibt eine Wahrscheinlichkeit von ca. 4 x 10⁻⁴ dafür, daß sich ein Rhodamin-110 Molekül zu einem gegebenen Zeitpunkt im beobachteten Probenraum befindet.

Ferner können auch höher konzentrierte Lösungen untersucht werden, wenn das Fluoreszenzverhalten des in einer solchen Lösung stark verdünnt vorliegenden Zielpartikels durch die in höherer Konzentration vorliegenden Partikel beeinflußt wird. Der Einfluß geschieht dabei durch lokale, molekulare Wechselwirkungen und kann sich sowohl auf die Fluoreszenzlebensdauer als auch auf andere Charakteristiken des Fluoreszenzverhaltens, wie die Intensität der Fluoreszenz, das Depolarisationsverhalten oder ähnliches, auswirken. Das Depolarisationsverhalten kann sich z.B. aufgrund einer Bindung zwischen den Zielpartikeln und den höher konzentriert vorliegenden Partikeln ändern.

Eine Bestimmung des Fluoreszenzverhaltens der Zielpartikel erlaubt unter diesen Umständen Rückschlüsse auf die höher konzentriert vorliegenden Partikel. Die Zielpartikel können dabei so stark verdünnt sein, daß sie in der Regel einzeln im Probenraum vorhanden sind.

Im folgenden wird erneut Fig. 3 betrachtet. Die relativ kleinen zeitlichen Abstände zwischen der detektierten Photonen bei der Ereignisnummer von ca. 1.900 bzw. die entsprechende relativ hohe Dichte der Detektionszeitpunkte, d.h. die hohe Detektionsrate von Photonen, weisen, wie bereits erklärt, auf das Vorliegen eines einzelnen Zielpartikels im Probenmedium hin. Für eine mehr quantitative Betrachtung können die in R. A. Keller, Applied Spectroscopy, Band 50 Nr. 7 (1996), Seiten 12A bis 32A beschriebenen Mittelungsund Schwellensetzungsverfahren verwendet werden.

Es kann aber auch jeder aus den Histogrammen bestimmbare Parameter, etwa der Amplitudenanteil einer bestimmten Fluoreszenzlebensdauer, z.B. über einfache Schwellensetzungsverfahren zur Entscheidung herangezogen werden, ob ein einzelnes Zielpartikel im Probenraum vorliegt.

Ferner kann ein statistisches Verfahren verwendet werden, das die Hypothese testet, ob das beobachtete Signal von reinem Hintergrundrauschen herrühren kann. Dazu wird die Wahrscheinlichkeit berechnet, in so kurzen zeitlichen Abständen eine Reihe von Photonen zu beobachten, und zwar unter der Annahme, daß sie von Hintergrundrauschen bzw. unverzögertem Streulicht herrühren. Sinkt diese Wahrscheinlichkeit unter ein vorgegebenes Maß, so liegt mit entsprechender Signifikanz mindestens ein fluoreszierendes Molekül bzw. ein Zielpartikel im Fokus vor.

Wählt man, wie es in den Figuren 3 bis 6 geschehen ist, die einem einzeln vorliegenden Zielpartikel zugeordneten Photonen aus und erstellt ein Histogramm, wie in Fig. 6, so kann die Fluoreszenzlebensdauer für ein einzelnes Zielpartikel bestimmt werden. Alternativ kann das beobachtete Fluoreszenzabklingverhalten mit vorbekannten Daten für bestimmte Arten von Zielpartikeln verglichen werden, und es kann entschieden werden, welche der bekannten Arten von Zielpartikeln in diesem Falle vorliegt. Dazu eignen sich insbesondere Algorithmen, die von M. Köllner in Applied Optics Band 32 (6) (1993), auf den Seiten 806 bis 820 beschrieben wurden. Auf diese Weise können einzelne Moleküle bzw. Zielpartikel identifiziert werden.

Die Anordnungen zur Datenerfassung und die zugehörigen Auswerteverfahren eignen sich damit für die in der DE 42 10 970 C2 beschriebenen Verfahren zur optischen qualitativen und quantitativen Erfassung von Biomolekülen etc. mittels Laserspektroskopie. Dies ist besonders dann der Fall, wenn die hier beschriebenen Anordnungen und Verfahren auf einzeln vorliegende Zielpartikel angewandt werden.

Ferner kann eines der soeben beschriebenen Auswerteverfahren dazu verwendet werden, festzustellen, ob ein einzelnes Zielpartikel im beobachteten Probenraum vorliegt. Die Daten werden danach nur dann einer Auswertung zugeführt, wenn die entsprechenden Algorithmen zu der Entscheidung geführt haben, daß mit hoher Wahrscheinlichkeit ein einzelnes Zielpartikel im Probenraum vorlag. Ein solches Vorgehen stellt eine gleitende Vorauswertung dar.

Ebensogut kann der Amplitudenanteil des unverzögerten Streulichts zum gesamten Streulicht dazu verwendet werden, die Daten für eine weitere Auswertung vorzufiltern.

Insbesondere kann mit Hilfe der vorgefilterten Signale in effizienter Weise die Autokorrelationsfunktion der detektierten Photonen für jeweils einzeln vorliegende Zielpartikel berechnet werden. Dies kann dadurch erreicht werden, daß die Autokorrelationsfunktion nur mit Hilfe der Photonen aufgebaut wird, die zu Zeiten detektiert wurden, als sich mit vorgegebener Wahrscheinlichkeit ein einzelnes Zielpartikel im Probenraum befand. Solche Photonen, die dem allgemeinen unverzögerten Streulicht und sonstigem Hintergrund zugeordnet wurden, werden nicht zur Berechnung herangezogen. Dies führt zu einer effektiven Streulichtunterdrückung und zu einer entsprechenden Verbesserung in der Genauigkeit und Amplitude der berechneten Autokorrelationsfunktion, da die Amplitude der Autokorrelationsfunktion vom Signal-Rausch-Verhältnis der der Autokorrelation zugrunde liegenden Funktion abhängt. Je schlechter dieses ist, desto geringer ist auch die Amplitude der Autokorrelationsfunktion (vgl. D.E. Koppel, Physical Review A, 1974, Heft 10, S. 1938).

Bei ausschließlicher Verwendung der Information, die in den Detektionszeitpunkten der Photonen vorliegt, unter Verzicht auf die Information aus den Verzögerungszeiten, kann auch eine Korrelationsfunktion allein für die Detektionszeitpunkte von einzelnen Zielpartikeln zugeordneten Photonen berechnet werden, und nicht für daraus bestimmte Parameter.

Dies führt gegenüber herkömmlicher FCS, wie oben bereits erläutert, zu einer deutlichen Verbesserung des Signal-Rausch-Verhältnisses. Die Korrelationsfunktion kann dabei selektiv für die einzelnen Zielpartikel berechnet werden.

Für diese Anwendung kann beliebiges Licht verwendet werden, insbesondere kontinuierliches Licht, das nicht moduliert ist.

Zur Beurteilung der Frage, ob ein Zielpartikel einzeln im Probenraum vorliegt, kann erfindungsgemäß eine relativ hohe zeitliche Dichte oder können relativ kurze zeitliche Abstände zwischen den Detektionszeitpunkten der aufeinanderfolgend detektierten Photonen herangezogen werden. Wie bereits beschrieben, kann dies in Fig. 3 um die Ereignisnummer 1.900 in Verbindung mit Fig. 4 deutlich erkannt werden. Die "Täler" in Fig. 3 erscheinen in Fig. 4 als Peaks, die allgemein auch als "Burst" bezeichnet werden.

Ein Vergleich des relativ kurzen zeitlichen Abstands zwischen den Detektionszeitpunkten der aufeinanderfolgend detektierten Photonen um die Ereignisnummer 1.100 in Fig. 3 mit dem Tal in der Kurve um die Ereignisnummer 1.900 zeigt, daß die Täler sehr unterschiedliche Dauern haben bzw. aus sehr unterschiedlich vielen detektierten Photonen sich ergeben. Um zu beurteilen, ob ein solches Tal durch eine zufällige Schwankung des Hintergrundsignals hervorgerufen wurde, oder auf ein einzeln vorliegendes Zielpartikel zurückzuführen ist, eignen sich die oben erwähnten statistischen Kriterien, die das Tal um die Ereignisnummer 1.100 als von sonstigem gestreutem Licht herrührend identifizieren würden.

Für eine sinnvolle Beurteilung, ob eine relativ hohe zeitliche Dichte der Detektionszeitpunkte der Photonen auf das Vorliegen eines Zielpartikels hinweist eignet sich daher auch eine Beurteilung der zeitlichen Dauer eines Bursts.

Im Rahmen des Erfindungsgedankens sind zahlreiche Abwandlungen möglich. Grundsätzlich kann zum Feststellen des Vorhandseins eines einzelnen Zielpartikels das Über- bzw. Unterschreiten sowohl eines Parameters (vgl. Fig. 7) als auch der Anzahl der pro Zeiteinheit erfaßten Detektionszeitpunkte (Intensität) herangezogen werden (vgl. Fig. 4). Es ergeben sich somit grundsätzlich zwei Möglichkeiten für Schwellensetzungen. Außerdem können jeweils entweder eine Korrelationsfunktion eines Parameters oder eine Korrelationsfunktion der Intensität berechnet werden. Aus den zwei Möglichkeiten der Schwellensetzung und den zwei Möglichkeiten, Korrelationsfunktionen zu berechnen, ergeben sich zusammen vier Kombinationsmöglichkeiten. So kann:
1. eine Schwelle für die Intensität vorgegeben und anschließend eine Korrelationsfunktion der Intensität berechnet werden.
2. eine Schwelle für die Intensität vorgegeben und anschließend eine Korrelationsfunktion eines Parameters berechnet werden.
3. eine Schwelle für einen Parameterwert vorgegeben und anschließend eine Korrelationsfunktion der Intensität berechnet werden.
4. eine Schwelle für einen Parameterwert vorgegeben und anschließend eine Korrelationsfunktion desselben oder eines anderen Parameters berechnet werden.

Lichtquelle, Detektionseinrichtung und Auswerteeinrichtung müssen zu den einzelnen Möglichkeiten, wie beschrieben, jeweils geeignet gewählt werden.

Somit ist es mit dem erfindungsgemäßen Verfahren und der erfindungsgemäßen Anordnung erstmals möglich, FCS auch bei geringsten Konzentrationen mit gutem Signal-Rausch-Verhältnis bzw. selektiv für ausgewählte Zielpartikel durchzuführen.

## Patentansprüche

1. Verfahren zum Bestimmen von Eigenschaften von Zielpartikeln eines Probenmediums, wobei:
a) das Probenmedium mit periodisch moduliertem Licht bestrahlt wird;
b) im Probenmedium gestreutes Licht in Form von einzelnen Photonen von einer Detektionseinrichtung detektiert wird;
c) der zeitliche Abstand zwischen dem Detektionszeitpunkt jedes detektierten Photons und einem in der zugehörigen Periode des bestrahlenden Lichts liegenden Referenzzeitpunkt als Verzögerungszeit registriert wird;
d) zusätzlich zur Bestimmung der Verzögerungszeit jedes detektierten Photons für jedes detektierte Photon der Detektionszeitpunkt registriert wird;
e) aus den Verzögerungszeiten jeweils einer Anzahl unmittelbar aufeinanderfolgend detektierter Photonen wenigstens ein Parameter bestimmt wird;
f) mittels der Detektionszeitpunkte der jeweiligen Anzahl unmittelbar aufeinanderfolgend detektierter Photonen ein Zeitwert bestimmt wird;
g) mittels des wenigstens einen Parameters und des Zeitwerts der jeweiligen Anzahl unmittelbar aufeinanderfolgend detektierter Photonen wenigstens ein Parameter-Zeit-Wertepaar bestimmt wird;
h) aus den Parameter-Zeit-Wertepaaren mehrerer aufeinanderfolgender Anzahlen wenigstens eine Parameter-Zeit-Funktion bestimmt wird;
i) eine Korrelationsfunktion der wenigstens einen Parameter-Zeit-Funktion berechnet wird; und
j) die Eigenschaften der Zielpartikel mit Hilfe der Korrelationsfunktion bestimmt werden.

2. Verfahren nach Anspruch 1, **dadurch gekennzeichnet, daß** das periodisch modulierte Licht pulsförmig moduliert ist.

3. Verfahren nach Anspruch 1 oder 2, **dadurch gekennzeichnet,**
**daß** das im Probenmedium gestreute Licht von mehr als einem Detektor detektiert wird; und
**daß** jeweils wenigstens eine Parameter-Zeit-Funktion für die von jedem Detektor detektierten Photonen getrennt berechnet wird.

4. Verfahren nach Anspruch 3, **dadurch gekennzeichnet, daß** als Korrelationsfunktion eine Kreuzkorrelationsfunktion mit Hilfe der Parameter-Zeit-Funktionen verschiedener Detektoren berechnet wird.

5. Verfahren nach einem der Ansprüche 1 bis 4, **dadurch gekennzeichnet,**
**daß** für jeweils eine vorgegebene Anzahl von aufeinanderfolgend detektierten Photonen des gestreuten Lichts oder für ein vorgegebenes Zeitintervall ein Histogramm der Verzögerungszeiten erstellt wird; und
**daß** aus dem Histogramm mit Hilfe von effizienten statistischen Verfahren Parameter des gestreuten Lichts bestimmt werden.

6. Verfahren nach einem der Ansprüche 1 bis 5, **dadurch gekennzeichnet, daß** als Zielpartikel fluoreszenzfähige Moleküle oder Gruppen (Fluorophore) aufweisende Partikel gewählt werden.

7. Verfahren nach Anspruch 6, **dadurch gekennzeichnet, daß** bei der Bestimmung des wenigstens einen Parameters des gestreuten Lichts berücksichtigt wird, daß sich das detektierte gestreute Licht wenigstens aus Fluoreszenzlicht der Zielpartikel und sonstigem gestreuten Licht anteilig zusammensetzt; und
daß als Parameter ein Anteil des gestreuten Lichts an der Gesamtamplitude bestimmt wird.

8. Verfahren nach Anspruch 7, **dadurch gekennzeichnet, daß** als Parameter eine Fluoreszenzlebensdauer bestimmt wird.

9. Verfahren nach Anspruch 7 oder 8, **dadurch gekennzeichnet,**
**daß** zur Bestimmung der Amplitudenanteile der einzelnen Anteile des gestreuten Lichts vorgegebene Informationen über die Zusammensetzung des Fluoreszenzlichts der Zielpartikel aus einer Überlagerung mehrerer einzelner exponentieller Abfälle, die sich durch spezifische Fluoreszenzlebensdauern beschreiben lassen, verarbeitet wird; und
**daß** als Parameter die Amplitudenanteile und/oder die Fluoreszenzlebensdauern der einzelnen exponentiellen Abfälle bestimmt werden.

10. Verfahren nach Anspruch 9, **dadurch gekennzeichnet, daß** bei der Bestimmung der Amplitudenanteile der einzelnen exponentiellen Abfälle Informationen über die typischen Fluoreszenzlebensdauern der Zielpartikel verwendet werden.

11. Verfahren nach einem der Ansprüche 7 bis 10, **dadurch gekennzeichnet, daß** die Amplitudenanteile des von den jeweiligen Zielpartikeln gestreuten Lichts mit Hilfe von Informationen über das Rotations- oder Fluoreszenz-Depolarisationsverhalten der Zielpartikel bestimmt werden.

12. Verfahren nach einem der Ansprüche 7 bis 11, **dadurch gekennzeichnet,**
**daß** als Zielpartikel wenigstens zwei jeweils Fluorophore aufweisende Partikel verwendet werden, wobei zwischen den jeweiligen Fluorophoren ein resonanter Energietransfer auftritt;
**daß** als Parameter die Amplitudenanteile des von den jeweiligen Fluorophoren gestreuten Lichts bestimmt werden; und
**daß** als Korrelationsfunktion eine Autokorrelationsfunktion der Amplitudenanteile des von den jeweiligen Fluorophoren gestreuten Lichts bestimmt wird.

13. Verfahren nach einem der Ansprüche 1 bis 12, **dadurch gekennzeichnet,**
**daß** die Dichte der Zielpartikel so gewählt wird, daß sich im Mittel weniger als ein Zielpartikel in einem beobachteten Volumenelement des Probenmediums (Probenraum) befindet; und
**daß** die Korrelationsfunktion ausschließlich aus Abschnitten der Parameter-Zeit-Funktion berechnet wird, die einzelnen Zielpartikeln zugeordnet sind.

14. Verfahren nach Anspruch 13, **dadurch gekennzeichnet, daß** ein Vorhandensein eines einzelnen Zielpartikels im Probenraum durch ein Überschreiten einer vorgegebenen Schwelle durch einen vorgegebenen Parameter des gestreuten Lichts festgestellt wird.

15. Verfahren zum Bestimmen von Eigenschaften von Zielpartikeln eines Probenmediums, wobei:
a) das Probenmedium mit Licht bestrahlt wird;
b) im Probenmedium gestreutes Licht in Form von einzelnen Photonen von einer Detektionseinrichtung detektiert wird;
c) der Detektionszeitpunkt jedes detektierten Photons erfaßt wird;
d) die Dichte der Zielpartikel so gewählt wird, daß sich im Mittel weniger als ein Zielpartikel in einem beobachteten Volumenelement des Probenmediums (Probenraum) befindet;
e ) durch Bewerten der erfaßten Detektionszeitpunkte Zeitintervalle bestimmt werden, in denen Zielpartikel einzeln im Probenraum vorlagen;
f) aus den in einem aus den Zeitintervallen ausgewählten Zeitintervall erfaßten Detektionszeitpunkten wenigstens eine auf das ausgewählte Zeitintervall begrenzte, zeitabhängige Funktion gebildet wird, die eine Anzahl der pro vorgegebener Zeiteinheit erfaßten Detektionszeitpunkte angibt, wobei die vorgegebene Zeiteinheit wesentlich kürzer als das ausgewählte Zeitintervall gewählt wird;
g) eine Korrelationsfunktion der zeitabhängigen Funktion berechnet wird; und
h) die Eigenschaften der Zielpartikel mit Hilfe der Korrelationsfunktion bestimmt werden.

16. Verfahren nach Anspruch 15, **dadurch gekennzeichnet, daß** das Absinken eines Mittelwerts der zeitlichen Abstände zwischen den Detektionszeitpunkten von aufeinanderfolgend detektierten Photonen über eine vorgegebene Anzahl von aufeinanderfolgend detektierten Photonen unter einen vorgegebenen Wert als Entscheidungskriterium für das Vorliegen eines einzelnen Zielpartikels im Probenraum verwendet wird.

17. Verfahren nach Anspruch 15, **dadurch gekennzeichnet, daß** das Absinken eines Mittelwerts der zeitlichen Abstände zwischen den Detektionszeitpunkten von aufeinanderfolgend detektierten Photonen über ein vorgegebenes Zeitintervall unter einen vorgegebenen Wert als Entscheidungskriterium für das Vorliegen eines einzelnen Zielpartikels im Probenraum verwendet wird.

18. Anordnung zur Durchführung des Verfahrens nach Anspruch 1 mit:
a) wenigstens einer Lichtquelle (20), die moduliertes Licht mit einer vorgegebenen Periodendauer aussendet;
b) einem Probenraum, in welchem Partikel eines Probenmediums mit dem Licht aus der Lichtquelle bestrahlt werden;
c) einer Einrichtung (8) zur Detektion von einzelnen Photonen aus dem Probenraum, die derart ausgebildet ist, daß sie bei Erfassen eines Photons aus dem Probenraum einen Impuls an einem ersten Ausgang (11) ausgibt und den zeitlichen Abstand zwischen dem Detektionszeitpunkt jedes Photons und einem in der zugehörigen Periode des bestrahlenden Lichts liegenden Referenzzeitpunkt als Verzögerungszeit bestimmt und in Form von Digitaldaten an einem zweiten Ausgang (12) ausgibt; und
d) einer mit dem ersten und zweiten Ausgang der Detektionseinrichtung gekoppelten Auswerteeinrichtung zum Aufnehmen und Auswerten der Digitaldaten;
**dadurch gekennzeichnet**,
e) daß die Auswerteeinrichtung eine mit dem zweiten Ausgang gekoppelte Recheneinheit (27), zwei jeweils Startund Stopeingänge aufweisende Zähler (17, 18) und eine die Start- und Stopeingänge der Zähler mit dem ersten Ausgang (11) der Detektionseinrichtung koppelnde Umschalteinrichtung (24) aufweist,
f) wobei die Umschalteinrichtung die Zähler bei jedem von der Detektionseinrichtung ausgegebenen Impuls gegensinnig ein- und ausschaltet, so daß der eine Zähler zählt, während der andere Zähler gestoppt ist und/oder den Zählerstand ausgibt, wobei der Takt der Zähler von einer Taktgebereinrichtung (25) vorgegeben ist; und
g) daß die Zählerstände zur Bestimmung der Eigenschaften der Zielpartikel an die Recheneinheit (27) übertragbar sind.

19. Anordnung nach Anspruch 18, **dadurch gekennzeichnet, daß** die Zähler (17, 18) derart geschaltet sind, daß sie nach dem Stoppen und vor einem erneuten Starten zurückgesetzt werden.

20. Anordnung nach Anspruch 18 oder 19, **dadurch gekennzeichnet, daß** die Auswerteeinrichtung derart ausgebildet ist, daß sie bei Empfang eines elektrischen Impulses von dem ersten Ausgang (11) die von der Detektionseinrichtung (8) ausgegebenen Digitaldaten in Zuordnung zu einem Zählerstand der Zähler (17, 18) speichern kann.

21. Anordnung nach einem dem Ansprüche 18 bis 20, **dadurch gekennzeichnet, daß** den Zählern (17, 18) wenigstens ein Zwischenspeicher (26) nachgeschaltet ist, der dazu dient, den Zählerstand bzw. die Zählerstände vor dem Übertragen an die Recheneinheit (27) zu speichern.

22. Anordnung nach einem der Ansprüche 18 bis 21, **dadurch gekennzeichnet,**
**daß** die Recheneinheit (27) einen Rechner aufweist, der eine digitale Ein- und Ausgabekarte (28) zur Aufnahme der Digitaldaten aufweist; und
**daß** die Karte zur Datenaufnahme durch die Impulse (11) der Detektionseinrichtung (8) triggerbar ist.

23. Anordnung nach einem der Ansprüche 18 bis 22, **dadurch gekennzeichnet, daß** die Detektionseinrichtung (8) mehr als einen Detektor aufweist.

24. Anordnung nach einem der Ansprüche 18 bis 23, **dadurch gekennzeichnet, daß** die Detektionseinrichtung (8) eine optische Anordnung in Form eines konfokalen Mikroskops oder eines Nahfeld-Mikroskops umfaßt.

## Claims

1. A method of determining properties of target particles in a sample medium wherein:
a) the sample medium is irradiated with periodically modulated light;
b) light in the form of individual photons scattered in the sample medium is detected by a detection device;
c) the time interval between the time of detection of each detected photon and a reference time within the associated period of the irradiating light is recorded as the delay time;
d) in addition to determining the delay time of each detected photon, the time of detection of each detected photon is recorded;
e) at least one parameter is determined from the delay times of a respective number of immediately successively detected photons;
f) a time value is determined via the times when the respective number of immediately successively detected photons were detected;
g) at least one pair of parameter and time values is determined via the at least one parameter and the time value of the respective number of immediately successively d etected photons;
h) at least one parameter-time function is determined from the pairs of parameter and time values of a plurality of successive numbers;
i) a correlation function of the at least one parameter-time function is calculated, and
j) the properties of the target particles are determined by means of the correlation function.

2. A method according to claim 1, **characterised in that** the periodically modulated light is modulated in pulses.

3. A method according to claim 1 or 2, **characterised in that** the light scattered in the sample medium is detected by more than one detector; and
**in that** in each case at least one parameter-time function for the photons detected by each detector is calculated separately.

4. A method according to claim 3, **characterised in that** the correlation function is a cross-correlation function calculated by using the parameter-time functions of various detectors.

5. A method according to any of claims 1 to 4, **characterised in that** a histogram of the delay times is prepared for each predetermined number of successively detected photons of the scattered light or for a predetermined time interval, and
**in that** parameters of the scattered light are determined from the histogram by efficient statistical methods.

6. A method according to any of claims 1 to 5, **characterised in that** the target particles are molecules capable of fluorescence or particles comprising groups (fluorophores).

7. A method according to claim 6, **characterised in that** the at least one parameter of the scattered light is determined allowing for the fact that the detected scattered light is made up of at least fluorescent light from the target particles and from proportions of other scattered light and
**in that** the parameter determined is the proportion of the scattered light to the total amplitude.

8. A method according to claim 7, **characterised in that** the parameter determined is a fluorescence life.

9. A method according to claim 7 or 8, **characterised in that** in order to determine the proportional amplitudes of the individual components of the scattered light, predetermined information about the composition of the fluorescent light from the target particles is processed by superposing a number of individual exponential decays which can be described by specific fluorescence lives, and
**in that** the parameters determined are the proportional amplitudes and/or the fluorescence lives of the individual exponential decays.

10. A method according to claim 9, **characterised in that** information about the typical fluorescence lives of the target particles is used in determining the proportional amplitudes of the individual exponential decays.

11. A method according to any of claims 7 to 10, **characterised in that** the proportional amplitudes of the light scattered by the respective target particles are determined by using information about the rotation or fluorescence depolarisation behaviour of the target particles.

12. A method according to any of claims 7 to 11, **characterised in that** the target particles used are at least two particles each comprising fluorophores, a resonant energy transfer occurring between the respective fluorophores;
**in that** the parameters determined are the proportional amplitudes of the light scattered by the respective fluorophores, and
**in that** the correlation function determined is an autocorrelation function of the proportional amplitudes of the light scattered by the respective fluorophores.

13. A method according to any of claims 1 to 12, **characterised in that** the density of the target particles is so chosen that on average less than one target particle is present in an observed volume element of the sample medium (sample space) and
**in that** the correlation function is calculated exclusively from portions of the parameter-time function associated with individual target particles.

14. A method according to claim 13, **characterised in that** the presence of an individual target particle in the sample space is ascertained when a predetermined threshold is exceeded by a predetermined parameter of the scattered light.

15. A method of determining properties of target particles in a sample medium, wherein
a) the sample medium is irradiated with light;
b) light in the form of individual photons scattered in the sample medium is detected by a detection device;
c) the time of detection of each detected photon is measured;
d) the density of the target particles is so chosen that on average less than one target particle is present in an observed volume element of the sample medium (sample space);
e) the measured detection times are evaluated so as to determine time intervals during which target particles were individually present in the sample space;
f) out of the times of detection measured in a time interval selected from among the time intervals, at least one time-dependent function limited to the selected time interval is formed and indicates a number of times of detection measured per predetermined unit time, the predetermined unit time being considerably shorter than the selected time interval;
g) a correlation function of the time-dependent function is calculated, and
h) the properties of the target particles are determined by using the correlation function.

16. A method according to claim 15, **characterised in that** when the average value of the time intervals between the times of detection of successively detected photons over a pedetermined number thereof falls below a predetermined value, this is used as a decision criterion for the presence of an individual target particle in the sample space.

17. A method according to claim 15, **characterised in that** when an average value of the time intervals between the times of detection of successively detected photons over a predetermined time interval falls below a predetermined value, this is used as a decision criterion for the presence of an individual target particle in the sample space.

18. An arrangement for working the method according to claim 1 comprising:
a) at least one light source (20) which transmits modulated light having a predetermined period;
b) a sample space in which particles of a sample medium are irradiated with the light from the light source;
c) a device (8) for detecting individual photons from the sample space and constructed so that when a photon from the sample space is detected it delivers a pulse at a first output (11) and determines and defines the delay time as the time interval between the time of detection of each photon and a reference time within the associated period of the irradiating light and delivers the delay time in the form of digital data at a second output (12), and
d) an evaluation device coupled to the first and second output of the detection device for receiving and evaluating the digital data;
**characterised in that**
e) the evaluating device comprises a computer unit (27) coupled to the second output, two counters (17, 18) each having start and stop inputs, and a change-over device (24) which couples the start and stop inputs of the counters to the first output (11) of the detection device,
f) wherein the change-over device switches the counters on and off in opposite directions at each pulse output by the detection device, so that one counter counts while the other counter is stopped and/or delivers the count, the cycle time of the counters being predetermined by a clock generator device (25), and
g) the counts are transferable to the computer unit (27) in order to determine the properties of the target particles.

19. An arrangement according to claim 18, **characterised in that** the counters (17, 18) are so connected that they are reset after stopping and before restarting.

20. An arrangement according to claim 18 or 19, **characterised in that** the evaluating device is so constructed that on receipt of an electric pulse from the first output (11) it can store the digital data output by the detection device (8) and associate them with a count of the counters (17, 18).

21. An arrangement according to any of claims 18 to 20, **characterised in that** the counters (17, 18) are followed by at least one intermediate memory (26) for storing the count or counts before transfer to the computer unit (27).

22. An arrangement according to any of claims 18 to 21, **characterised in that** the computer unit (27) comprises a computer which has a digital input and output card (28) for receiving the digital data, and
**in that** the data-receiving card is triggerable by the pulses (11) from the detection device (8).

23. An arrangement according to any of claims 18 to 22, **characterised in that** the detection device (8) comprises more than one detector.

24. An arrangement according to any of claims 18 to 23, **characterised in that** the detection device (8) comprises an optical arrangement in the form of a confocal microscope or a near-field microscope.

## Revendications

1. Procédé de détermination de propriétés de particules cibles d'un milieu échantillon, dans lequel :
a) le milieu échantillon est irradié par de la lumière modulée périodiquement,
b) la lumière diffusée dans le milieu échantillon est détectée sous forme de photons individuels par un dispositif de détection,
c) l'intervalle de temps entre l'instant de détection de chaque photon détecté et un instant de référence situé dans la période associée de la lumière irradiante est enregistré comme retard,
d) en plus de la détermination du retard de chaque photon détecté, pour chaque photon détecté est enregistré l'instant de détection,
e) à partir des retards chaque fois d'un certain nombre de photons détectés immédiatement successivement est déterminé au moins un paramètre,
f) au moyen des instants de détection dudit nombre de photons détectés immédiatement successivement est déterminée une valeur de temps,
g) au moyen du ou des paramètres et de la valeur de temps dudit nombre de photons détectés immédiatement successivement est déterminée au moins une paire paramètre(s)-valeur de temps,
h) à partir des paires paramètre(s)-valeur de temps de plusieurs nombres successifs est déterminée au moins une fonction paramètre(s)-temps,
i) une fonction de corrélation de la ou des fonctions paramètre(s)-temps est calculée, et
j) les propriétés des particules cibles sont déterminées à l'aide de la fonction de corrélation.

2. Procédé selon la revendication 1, **caractérisé par le fait que** la lumière modulée périodiquement est modulée sous forme d'impulsions.

3. Procédé selon l'une des revendications 1 et 2, **caractérisé par le fait**
**que** la lumière diffusée dans le milieu échantillon est détectée par plusieurs détecteurs, et
**que** chaque fois au moins une fonction paramètre(s)-temps est calculée séparément pour les photons détectés par chaque détecteur.

4. Procédé selon la revendication 3, **caractérisé par le fait que** comme fonction de corrélation, une fonction d'intercorrélation est calculée à l'aide des fonctions paramètre(s)-temps de différents détecteurs.

5. Procédé selon l'une des revendications 1 à 4, **caractérisé par le fait**
**que** pour chaque fois un nombre fixé de photons détectés successivement de la lumière diffusée ou pour un intervalle de temps fixé est établi un histogramme des retards, et
**qu'**à partir de l'histogramme sont déterminés à l'aide de méthodes statistiques efficaces des paramètres de la lumière diffusée.

6. Procédé selon l'une des revendications 1 à 5, **caractérisé par le fait que** comme particules cibles sont choisies des particules qui présentent des molécules ou des groupes (fluorophores) pouvant entrer en fluorescence.

7. Procédé selon la revendication 6, **caractérisé par le fait**
**que** lors de la détermination du ou des paramètres de la lumière diffusée, il est tenu compte de ce que la lumière diffusée détectée se compose au moins d'une part de lumière de fluorescence des particules cibles et d'une part de lumière diffusée autre, et
**que** comme paramètre est déterminée une part de la lumière diffusée dans l'amplitude totale.

8. Procédé selon la revendication 7, **caractérisé par le fait que** comme paramètre est déterminée une durée de vie de fluorescence.

9. Procédé selon l'une des revendications 7 et 8, **caractérisé par le fait**
**que** pour la détermination des parts d'amplitude des différentes parts de lumière diffusée sont traitées des informations fixées concernant la composition de la lumière fluorescente des particules cibles résultant d'une superposition de plusieurs décroissances exponentielles individuelles qui peuvent être représentées par des durées de vie de fluorescence spécifiques, et
**que** comme paramètres sont déterminées les parts d'amplitude et/ou les durées de vie de fluorescence des décroissances exponentielles individuelles.

10. Procédé selon la revendication 9, **caractérisé par le fait que** lors de la détermination des parts d'amplitude des décroissances exponentielles individuelles sont utilisées des informations concernant les durées de vie de fluorescence typiques des particules cibles.

11. Procédé selon l'une des revendications 7 à 10, **caractérisé par le fait que** les parts d'amplitude de la lumière diffusée par les particules cibles considérées sont déterminées à l'aide d'informations concernant le comportement des particules cibles en matière de rotation ou de dépolarisation de fluorescence.

12. Procédé selon l'une des revendications 7 à 11, **caractérisé par le fait**
**que** comme particules cibles sont utilisées au moins deux particules présentant chacune des fluorophores, entre les fluoropohores respectifs ayant lieu un transfert d'énergie en résonance,
**que** comme paramètre sont déterminées les parts d'amplitude de la lumière diffusée par les fluorophores respectifs, et
**que** comme fonction de corrélation est déterminée une fonction d'autocorrélation des parts d'amplitude de la lumière diffusée par les fluorophores respectifs.

13. Procédé selon l'une des revendications 1 à 12, **caractérisé par le fait**
**que** la densité de particules cibles est choisie de façon qu'en moyenne, moins d'une particule cible se trouve dans un élément de volume observé du milieu échantillon (espace échantillon), et
**que** la fonction de corrélation est calculée uniquement à partir de parties de la fonction paramètre(s)-temps qui sont associées à des particules cibles individuelles.

14. Procédé selon la revendication 13, **caractérisé par le fait que** la présence d'une particule cible individuelle dans l'espace échantillon est constatée au dépassement d'un seuil fixé par un paramètre fixé de la lumière diffusée.

15. Procédé de détermination de propriétés de particules cibles d'un milieu échantillon, dans lequel :
a) le milieu échantillon est irradié par de la lumière,
b) la lumière diffusée dans le milieu échantillon est détectée sous forme de photons individuels par un dispositif de détection,
c) l'instant de détection de chaque photon détecté est saisi,
d) la densité de particules est choisie de façon qu'en -moyenne, moins d'une particule cible se trouve dans un élément de volume observé du milieu échantillon (espace échantillon),
e) par évaluation des instants de détection saisis sont déterminés des intervalles de temps dans lesquels des particules cibles étaient présentes individuellement dans l'espace échantillon,
f) à partir des instants de détection saisis dans un intervalle de temps choisi parmi les intervalles de temps est formée au moins une fonction dépendant du temps et limitée à l'intervalle de temps choisi qui indique un nombre d'instants de détection saisis par unité de temps fixée, l'unité de temps fixée étant choisie beaucoup plus courte que l'intervalle de temps choisi,
g) une fonction de corrélation de la fonction dépendant du temps est calculée, et
h) les propriétés des particules cibles sont déterminées à l'aide de la fonction de corrélation.

16. Procédé selon la revendication 15, **caractérisé par le fait que** la descente au-dessous d'une valeur fixée, d'une valeur moyenne des intervalles de temps entre les instants de détection de photons détectés successivement, valeur moyenne sur un nombre fixé de photons détectés successivement, est utilisée comme critère de décision de la présence d'une particule cible individuelle dans l'espace échantillon.

17. Procédé selon la revendication 15, **caractérisé par le fait que** la descente au-dessous d'une valeur fixée, d'une valeur moyenne des intervalles de temps entre les instants de détection de photons détectés successivement, valeur moyenne sur un intervalle de temps fixé, est utilisée comme critère de décision de la présence d'une particule cible individuelle dans l'espace échantillon.

18. Ensemble pour la mise en oeuvre du procédé de la revendication 1, comportant :
a) au moins une source lumineuse (20) qui émet de la lumière modulée avec une période fixée,
b) un espace échantillon dans lequel des particules d'un milieu échantillon sont irradiées par la lumière émise par la source lumineuse,
c) un dispositif (8) pour la détection de photons individuels de l'espace échantillon qui est conçu de façon telle qu'en cas de saisie d'un photon de l'espace échantillon, il émet une impulsion à une première sortie (11) et détermine comme retard l'intervalle de temps entre l'instant de détection de chaque photon et un instant de référence situé dans la période associée de la lumière irradiante et l'émet sous forme de données numériques à une deuxième sortie (12), et
d) un dispositif d'exploitation relié à la première et la deuxième sorties du dispositif de détection et destiné à recevoir et exploiter les données numériques,
**caractérisé par** le fait
e) que le dispositif d'exploitation présente une unité de calcul (27) reliée à la deuxième entrée, deux compteurs (17, 18) présentant chacun des entrées de démarrage et d'arrêt, et un dispositif de commutation (24) reliant les entrées de démarrage et d'arrêt des compteurs à la première sortie (11) du dispositif de détection,
f) le dispositif de commutation mettant en marche et arrêtant les compteurs en sens contraire à chaque impulsion émise par le dispositif de détection, de sorte qu'un compteur compte pendant que l'autre est arrêté et/ou émet son état, le rythme des compteurs étant fixé par un dispositif générateur de rythme (25), et
g) que les états des compteurs peuvent être transmis à l'unité de calcul (27) pour la détermination des propriétés des particules cibles.

19. Ensemble selon la revendication 18, **caractérisé par le fait que** les compteurs (17, 18) sont montés de façon à être remis à zéro après l'arrêt et avant un nouveau démarrage.

20. Ensemble selon l'une des revendications 18 et 19, **caractérisé par le fait que** le dispositif d'exploitation est conçu de façon telle qu'à la réception d'une impulsion électrique de la première sortie (11), il puisse enregistrer les données numériques émises par le dispositif de détection (8) en correspondance avec un état des compteurs (17, 18).

21. Ensemble selon l'une des revendications 18 à 20, **caractérisé par le fait qu'**en aval des compteurs (17, 18) est montée au moins une mémoire tampon (26) qui sert à enregistrer l'état d'un compteur ou les états des compteurs avant la transmission à l'unité de calcul (27).

22. Ensemble selon l'une des revendications 18 à 21, **caractérisé par le fait**
**que** l'unité de calcul (27) présente un calculateur qui présente une carte numérique d'entrée et de sortie (28) destinée à recevoir les données numériques, et
**que** la carte peut, pour la réception de données, être déclenchée par les impulsions (11) du dispositif de détection (8).

23. Ensemble selon l'une des revendications 18 à 22, **caractérisé par le fait que** le dispositif de détection (8) présente plusieurs détecteurs.

24. Ensemble selon l'une des revendications 18 à 23, **caractérisé par le fait que** le dispositif de détection (8) comprend un dispositif optique formé d'un microscope confocal ou d'un microscope à champ proche.
